# EUROPEAN PATENT APPLICATION

(11) **EP 4 671 282 A1**
(43) Date of publication of application: **31.12.2025**
(21) Application number: 24759708.1
(22) Date of filing: 21.02.2024
(51) Int. Cl.: C07K 16/46, C07K 16/24, C07K 19/00, C12N 15/62, A61K 39/395, A61P 37/00

(54) **IL-36R BINDING PROTEIN AND MEDICINAL USE THEREOF**

(30) Priority: 21.02.2023 CN 202310148631
(71) Applicant: Jiangsu Hengrui Pharmaceuticals Co., Ltd., Lianyungang, Jiangsu 222047 (CN); Shanghai Shengdi Pharmaceutical Co., Ltd, Shanghai 201210 (CN)
(72) Inventor: RUN, Changqing, Shanghai 201210 (CN); SU, Lu, Shanghai 201210 (CN); CUI, Xueqin, Shanghai 201210 (CN); LIN, Yuan, Shanghai 201210 (CN); LIN, Kan, Shanghai 201210 (CN); LIAO, Cheng, Shanghai 201210 (CN)
(74) Representative: Dragotti & Associati S.P.A.
(86) International application number: PCT/CN2024/077938
(87) International publication number: WO 2024/175031

(57) **Abstract**

The present disclosure relates to an IL-36R binding protein and a medicinal use thereof. Specifically, the present disclosure relates to an IL-36R/IL-23 binding protein, an IL-36R binding protein, a method for treating inflammatory diseases and autoimmune diseases, and a related pharmaceutical use.

## Description

The present disclosure claims priority to Chinese Patent Application No. 202310148631.7, filed on February 21, 2023, which is incorporated herein by reference in its entirety.

### TECHNICAL FIELD

The present disclosure relates to the field of biopharmaceutics and particularly to an IL-36R-binding protein, a method for treating a cancer by using same, and related pharmaceutical use thereof.

### BACKGROUND

Interleukin-36 (IL-36) belongs to the IL-1 superfamily and includes three cytokines with pro-inflammatory functions: IL-36α, IL-36β, and IL-36γ. IL-36 is mainly secreted by epithelial cells and inflammatory immune cells in an inactivated full-length form. After IL-36 is cleaved by neutrophil-derived proteases (cathepsin G, elastases, etc.) at sites of inflammatory diseases, active fragments are released and further bind to receptors to exert downstream functions. An IL-36 receptor is a heterodimer consisting of IL-36R (also known as interleukin-1 receptor-like 2, IL1RL2, which is mainly expressed in epithelial cells and monocytes) and IL-1RAcP. IL-36 recruits IL-1RAcP after binding to IL-36R, and exerts downstream functions by activating signaling pathways such as MAPK and NF-κB. IL-36RA and IL-38 can bind to IL-36R and inhibit the recruitment of IL-1RAcP, thereby blocking the activation of downstream pathways by IL-36 (Elias M et al. JCI. 2021).

The activation of the IL-36 pathway mediates multiple biological processes: 1) promoting the proliferation of keratinocytes and inhibiting their differentiation; 2) promoting the release of various cytokines (including IL-6, TNFα, IL-8, CCL20, CXCL10, etc.); and 3) promoting the maturation and activation of dendritic cells (DCs) (Yuan ZC et al. Frontiers Immunol. 2019). Dysregulation of the IL-36 pathway is associated with a variety of autoimmune diseases, and upregulation of IL-36 expression is observed in skin lesions of many patients with inflammatory skin diseases (including psoriasis, hidradenitis suppurativa, lichen simplex chronicus, prurigo nodularis, pemphigus, etc.) and in the intestinal tracts of patients with inflammatory bowel diseases (ulcerative colitis and Crohn's disease) (Aquino TM et al. J Cutan Pathol. 2021). It has been reported in the literature that inactivating mutations of an inhibitory ligand IL-36RA are closely associated with the development of generalized pustular psoriasis (GPP). The proportion of patients with GPP carrying inactivating mutations of IL-36RA in China was about 46.8%-60.5% (Li X et al. J Dermatol Sci, 2014), and one study in Japan showed that 9/11 patients with GPP only and 2/20 patients with both GPP and psoriasis vulgaris carried inactivating mutations of IL-36RA (Sugiura K et al, J Invest Dermatol, 2013). In a preclinical IMQ-induced psoriasis model, blocking the IL-36 pathway can significantly reduce disease scores and inhibit downstream inflammatory disease gene expression (Mahil SK et al. Sci Trans Med, 2017).

Interleukin-23 (IL-23) belongs to the IL-12 family and is a heterodimer consisting of a p19 subunit and a p40 subunit. IL-23 is mainly secreted by activated macrophages and DCs. After binding to an IL-23R/IL-12Rβ1 heterodimeric receptor complex, IL-23 promotes the differentiation and phenotype maintenance of type 17 T cells and promotes the secretion of a variety of type 17 cytokines (e.g., IL-17A, IL-17F, and IL-22) by activating the JAK-STAT pathway (Elias M et al. JCI. 2021; Teng MWL et al. Nat Med. 2016). The IL-23/IL-17 pathway plays an important role in a variety of autoimmune diseases.

Although anti-IL-23 antibodies have relatively good efficacy in psoriasis, some patients still do not respond well to anti-IL-23 antibodies (Ghoreschi, K *et al, The Lancet,* 2021). The gene expression of the IL-36 pathway was significantly increased in the skin lesions in the patients with poor response to the IL-23 antibodies. IL-36 is directly involved in inflammatory skin diseases and immune cell recruitment. Targeting both IL-36 and IL-23 pathways can potentially improve the onset rate of anti-IL-23 antibodies and expand the population benefiting from them. In view of the unmet needs of patients during clinical treatment, the present disclosure provides a highly active therapeutic anti-IL-36R antibody that can specifically bind to IL-36R and has excellent antigen-binding specificity, affinity, and pharmacokinetic and pharmacodynamic properties that can be used for intervening in or treating a disease associated with the IL-36R signaling pathway, particularly an autoimmune disease. In addition, the present disclosure provides a therapeutic humanized anti-IL-36R/IL-23 bispecific antibody with a novel mechanism, which can specifically bind to both IL-36R and IL-23 and has excellent antigen-binding specificity, affinity, and pharmacokinetic and pharmacodynamic properties that can be used for intervening in or treating a disease associated with the IL-36R/IL-23 signaling pathways, particularly an autoimmune disease.

### SUMMARY

The present disclosure provides an IL-36R-binding protein, an IL-36R1IL-23-binding protein, a coding nucleic acid therefor, a vector thereof, a host cell thereof, and a pharmaceutical composition thereof, as well as a method for treating or preventing a disease by using same and related pharmaceutical use thereof.

### IL-36R-Binding Protein

The present disclosure provides an IL-36R-binding protein comprising an immunoglobulin single variable domain, wherein the immunoglobulin single variable domain comprises:
a CDR1, a CDR2, and a CDR3 from the amino acid sequence set forth in any one of SEQ ID NOs: 3 and 12-26, or
a CDR1, a CDR2, and a CDR3 from the amino acid sequence set forth in any one of SEQ ID NOs: 4 and 27-35,
wherein the CDR1, CDR2, and CDR3 are defined according to the Kabat, IMGT, Chothia, AbM, or Contact numbering scheme, for example, according to the Kabat numbering scheme.

In some embodiments, provided is an IL-36R-binding protein comprising any one of or any combination of the CDR1, CDR2, and CDR3 described above.

In some embodiments, provided is an IL-36R-binding protein comprising an immunoglobulin single variable domain, wherein the amino acid sequences of the CDR1, CDR2, and CDR3 of the immunoglobulin single variable domain are set forth in SEQ ID NOs: 5, 6, and 7, or SEQ ID NOs: 8, 9, and 10, respectively.

In some embodiments, the immunoglobulin single variable domain in the aforementioned IL-36R-binding protein is engineered by humanization, back mutation, avidity maturation, T cell epitope (TCE) removal/decreasing the number of TCEs, reduction of antibody deamidation, and/or reduction of antibody isomerization.

In some embodiments, the immunoglobulin single variable domain is obtained by TCE removal/decreasing the number of TCEs and has one or more changes in one or more CDRs, and the changes cause a reduction in the immunogenicity of the IL-36R-binding protein.

In some embodiments, the immunoglobulin single variable domain comprises one, more, or any combination of mutations in the amino acid residues at positions 1, 27, 28, 29, 30, 37, 44, 45, 47, 49, 71, 74, 76, 78, 94, and 103 (numbering according to the Kabat numbering scheme), e.g., one, more, or any combination of mutations selected from the group consisting of 1D, 27Y, 28S, 29Y, 30R, 37Y, 44Q, 45R, 47A, 49A, 71Q, 74A, 76T, 78V, 94A, and 103S, such as a combination of mutations in Table 3-1 of the present disclosure.

In some embodiments, the immunoglobulin single variable domain comprises one, more, or any combination of mutations in the amino acid residues at positions 27, 29, 37, 33, 45, 47, 49, 71, and 94 (numbering according to the Kabat numbering scheme), e.g., one, more, or any combination of mutations selected from the group consisting of 27N, 29Y, 37F, 44E, 45R, 47G, 49A, 71K, and 94A, such as a combination of mutations in Table 3-2 of the present disclosure.

In some embodiments, the immunoglobulin single variable domain comprises one, more, or any combination of mutations selected from the group consisting of F27N, F29Y, V37F, G44E, L45R, W47G, S49A, R71K, and R94A, such as a combination of mutations in Table 3-2 of the present disclosure.

In some embodiments, the amino acid sequence of the immunoglobulin single variable domain in the aforementioned IL-36R-binding protein is set forth in any one of SEQ ID NOs: 3 and 12-26 or has at least 80% or at least 90% sequence identity to any one of SEQ ID NOs: 3 and 12-26; in some embodiments, the amino acid sequence of the immunoglobulin single variable domain in the aforementioned IL-36R-binding protein is set forth in any one of SEQ ID NOs: 4 and 27-35 or has at least 80% or at least 90% sequence identity to any one of SEQ ID NOs: 4 and 27-35.

In the present disclosure, "at least 80% (sequence) identity" encompasses at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% (sequence) identity; "at least 90% (sequence) identity" encompasses at least 90%, at least 91%, at least 92%, at least 93%, at least 94%, at least 95%, at least 96%, at least 97%, at least 98%, at least 99%, or 100% (sequence) identity.

In some embodiments, the aforementioned IL-36R-binding protein comprises or is an antibody that specifically binds to a human IL-36R protein or a fragment thereof, or an antigen-binding fragment thereof. In some specific embodiments, the antibody or the antigen-binding fragment thereof is, for example, a camelid antibody, a chimeric antibody, a humanized antibody, or a fully human antibody, or an antigen-binding fragment thereof. In some specific embodiments, the antibody or the antigen-binding fragment thereof is, for example, a recombinant antibody or a fragment thereof. In some specific embodiments, the antibody or the antigen-binding fragment thereof is a linear antibody, a single-chain antibody, a nanobody, a peptibody, a domain antibody, or a multispecific antibody (a bispecific antibody, a diabody, a triabody, a tetrabody, a tandem di-scFv, or a tandem tri-scFv).

In some embodiments, the immunoglobulin single variable domain in the aforementioned IL-36R-binding protein is a single-domain antibody or VHH.

In some embodiments, the present disclosure provides an IL-36R-binding protein comprising one or more (e.g., 2, 3, 4, 5, 6, 7, or 8) of the aforementioned immunoglobulin single variable domains, wherein the immunoglobulin single variable domains may be identical or different and may form a dimeric or multimeric molecule.

In some embodiments, the aforementioned IL-36R-binding protein further comprises an Fc region of a human immunoglobulin; for example, the Fc region is an Fc region of human IgG1, IgG2, or IgG4. In some embodiments, the Fc region is an Fc region of human IgG4 comprising an S228P mutation, e.g., the Fc region is set forth in SEQ ID NO: 11 or has at least 80% or at least 90% sequence identity thereto. In some embodiments, the Fc region has increased stability, or comprises a mutation that makes the Fc region more stable than a wild-type Fc region. In some embodiments, the Fc region can cause the binding protein to form a dimeric molecule. In some embodiments, the Fc region can extend the *in vivo* half-life of the binding protein.

In some embodiments, in the aforementioned IL-36R-binding protein, the immunoglobulin single variable domain and the Fc region are linked, either directly or by a linker. The linker may be a non-functional amino acid sequence of 1-20 or more amino acids without a secondary or higher structure. For example, the linker is a flexible linker such as G₄S, GS, GAP, (G₄S)₂, (G₄S)₃, (G₄S)₄, (G₄S)₅, or ASGS. For example, the linker is (G₄S)₂.

In some embodiments, the IL-36R-binding protein of the present disclosure is an anti-IL-36R antibody or an antigen-binding fragment thereof, or a conjugate or fusion protein comprising the antibody or the antigen-binding fragment.

In some embodiments, the aforementioned IL-36R-binding protein has an activity selected from the group consisting of at least one of the following:
(a) binding to human IL-36R or an epitope thereof with a K_{D} value of ≤10⁻⁷;
(b) having the activity of inhibiting IL-17 secretion; preferably, inhibiting IL-17 secretion with an IC₅₀ value of less than 0.03 nM (e.g., less than 0.03 nM, less than 0.02 nM, less than 0.01 nM, or less), wherein in some embodiments, the IC₅₀ value is measured by the method in Example 5 of the present disclosure;
(c) having the activity of inhibiting proliferation of BaF3-IL-23R cells; preferably, inhibiting proliferation of BaF3-IL-23R cells with an IC₅₀ value of less than 0.5 nM (e.g., less than 0.5 nM, less than 0.4 nM, less than 0.3 nM, less than 0.2 nM, less than 0.1 nM, or less), wherein in some embodiments, the IC₅₀ value is measured by the method in Example 9;
(d) having the activity of inhibiting the STAT pathway; preferably, inhibiting the STAT3 pathway with an IC₅₀ value of less than 10 nM (e.g., less than 9 nM, less than 8 nM, or less), wherein in some embodiments, the IC₅₀ value is measured by the method in Example 9;
(e) having the activity of inhibiting a human IL-36 stimulating factor; preferably, inhibiting IL-8 secretion with an IC₅₀ value of less than 0.5 nM (e.g., less than 0.5 nM, less than 0.4 nM, less than 0.3 nM, less than 0.2 nM, less than 0.1 nM, or less), wherein in some embodiments, the IC₅₀ value is measured by the method in Example 8.

In some embodiments, the aforementioned IL-36R-binding protein of the present disclosure may bind to IL-36R with a K_{D} value of ≤1 × 10⁻⁷ M, e.g., ≤1 × 10⁻⁸ M, or ≤1 × 10⁻⁹ M, or ≤1 × 10⁻¹⁰ M.

In some embodiments, the aforementioned IL-36R-binding protein of the present disclosure encompasses a variant, wherein the variant comprises one or more (e.g., 1, 2, 3, 4, 5, 6, 7, 8, 9, or 10) amino acid mutations as compared to any one of SEQ ID NOs: 3 and 12-26 or any one of SEQ ID NOs: 4 and 27-35; the amino acid mutations may be conservative replacements, substitutions, or modifications, and/or deletions or additions that do not affect function; the amino acid mutations may occur in the CDRs and/or FRs. In some embodiments, provided is an anti-IL-36R antibody or an antigen-binding fragment thereof that binds to or competes with the immunoglobulin single variable domain in the aforementioned IL-36R-binding protein of the present disclosure for binding to the same epitope.

In some embodiments, provided is an anti-IL-36R antibody or an antigen-binding fragment thereof that blocks the binding of the immunoglobulin single variable domain in the aforementioned IL-36R-binding protein of the present disclosure to IL-36R (e.g., human IL-36R).

In some embodiments, provided is an anti-IL-36R antibody or an antigen-binding fragment thereof whose binding to IL-36R (e.g., human IL-36R) is blocked by the immunoglobulin single variable domain in the aforementioned IL-36R-binding protein of the present disclosure.

In some embodiments, provided is a protein or molecule comprising any one or more (e.g., 1, 2, 3, 4, 5, 6, 7, 8, 9, or 10) of the immunoglobulin single variable domains in the aforementioned IL-36R-binding proteins of the present disclosure, wherein the immunoglobulin single variable domains are identical or different. For example, the protein or molecule is a conjugate, and the conjugate may, for example, comprise any detectable label.

### IL-36R/IL-23-Binding Protein

The present disclosure provides an IL-36R/IL-23-binding protein that specifically binds to human IL-36R and/or a human IL-23 p19 subunit.

In some embodiments, provided is an IL-36R/IL-23-binding protein comprising a first antigen-binding domain that specifically binds to IL-36R, and a second antigen-binding domain that specifically binds to IL-23 (e.g., an IL-23 p19 subunit).

### Regarding the first antigen-binding domain that specifically binds to IL-36R:

In some embodiments, the first antigen-binding domain in the IL-36R/IL-23-binding protein comprises or is the immunoglobulin single variable domain in the aforementioned IL-36R-binding protein of the present disclosure.

In some specific embodiments, the immunoglobulin single variable domain comprises:
a CDR1, a CDR2, and a CDR3 from the amino acid sequence set forth in any one of SEQ ID NOs: 3 and 12-26, or
a CDR1, a CDR2, and a CDR3 from the amino acid sequence set forth in any one of SEQ ID NOs: 4 and 27-35,
wherein the CDR1, CDR2, and CDR3 are defined according to the Kabat, IMGT, Chothia, AbM, or Contact numbering scheme, for example, according to the Kabat numbering scheme.

In some specific embodiments, the immunoglobulin single variable domain comprises any one of or any combination of the CDR1, CDR2, and CDR3 described above.

In some specific embodiments, the amino acid sequences of the CDR1, CDR2, and CDR3 of the immunoglobulin single variable domain are set forth in SEQ ID NOs: 5, 6, and 7, or SEQ ID NOs: 8, 9, and 10, respectively.

In some specific embodiments, the immunoglobulin single variable domain in the aforementioned IL-36R-binding protein is engineered by humanization, back mutation, avidity maturation, T cell epitope (TCE) removal/decreasing the number of TCEs, reduction of antibody deamidation, and/or reduction of antibody isomerization.

In some specific embodiments, the immunoglobulin single variable domain is engineered by humanization. Heavy chain framework regions (FRs) of a human germline template used in the humanization are derived from IGHV3-23*04, IGHV3-74*01, and/or IGHJ1*01. In some embodiments, the FR1, FR2, and FR3 are derived from IGHV3-23*04, and the FR4 is derived from IGHJ1*01. In some embodiments, the FR1, FR2, and FR3 are derived from IGHV3-74*01, and the FR4 is derived from IGHJ1*01.

In some specific embodiments, the immunoglobulin single variable domain comprises one, more, or any combination of mutations in the amino acid residues at positions 1, 27, 28, 29, 30, 37, 44, 45, 47, 49, 71, 74, 76, 78, 94, and 103 (numbering according to the Kabat numbering scheme), e.g., one, more, or any combination of mutations selected from the group consisting of 1D, 27Y, 28S, 29Y, 30R, 37Y, 44Q, 45R, 47A, 49A, 71Q, 74A, 76T, 78V, 94A, and 103S, such as a combination of mutations in Table 3-1 of the present disclosure.

In some specific embodiments, the immunoglobulin single variable domain comprises one, more, or any combination of mutations in the amino acid residues at positions 27, 29, 37, 33, 45, 47, 49, 71, and 94 (numbering according to the Kabat numbering scheme), e.g., one, more, or any combination of mutations selected from the group consisting of 27N, 29Y, 37F, 44E, 45R, 47G, 49A, 71K, and 94A, such as a combination of mutations in Table 3-2 of the present disclosure.

In some specific embodiments, the amino acid sequence of the immunoglobulin single variable domain is set forth in any one of SEQ ID NOs: 3 and 12-26 or has at least 80% or at least 90% sequence identity to any one of SEQ ID NOs: 3 and 12-26; in some embodiments, the amino acid sequence of the immunoglobulin single variable domain in the aforementioned IL-36R-binding protein is set forth in any one of SEQ ID NOs: 4 and 27-35 or has at least 80% or at least 90% sequence identity to any one of SEQ ID NOs: 4 and 27-35.

In some specific embodiments, the immunoglobulin single variable domain is a single-domain antibody or VHH.

In some specific embodiments, the first antigen-binding domain that specifically binds to IL-36R comprises a fab or a VH and VL in BI655130 or H4H14706P2. WO2013074569A1 and WO2020018503A are incorporated in the present disclosure by reference in their entirety. In some specific embodiments, the first antigen-binding domain that specifically binds to IL-36R comprises a VH and a VL, wherein the VH comprises a HCDR1, a HCDR2, and a HCDR3 from SEQ ID NO: 36, and the VL comprises a LCDR1, a LCDR2, and a LCDR3 from SEQ ID NO: 37; for example, the VH is the VH from SEQ ID NO: 36 or has at least 80% or at least 90% identity thereto, and the VL is the VL from SEQ ID NO: 37 or has at least 80% or at least 90% identity thereto. In some specific embodiments, the VH comprises a HCDR1, a HCDR2, and a HCDR3 from SEQ ID NO: 38, and the VL comprises a LCDR1, a LCDR2, and a LCDR3 from SEQ ID NO: 39; for example, the VH is the VH from SEQ ID NO: 38 or has at least 80% or at least 90% identity thereto, and the VL is the VL from SEQ ID NO: 39 or has at least 80% or at least 90% identity thereto.

### Regarding the second antigen-binding domain that specifically binds to IL-23 (e.g., an IL-23 p19 subunit):

In some embodiments, the second antigen-binding domain comprises a heavy chain variable region (VH) and a light chain variable region (VL).

In some specific embodiments, in the second antigen-binding domain,
the VH comprises a HCDR1, a HCDR2, and a HCDR3 from the amino acid sequence set forth in SEQ ID NO: 40, and the VL comprises a LCDR1, a LCDR2, and a LCDR3 from the amino acid sequence set forth in SEQ ID NO: 41; or
the VH comprises a HCDR1, a HCDR2, and a HCDR3 from the amino acid sequence set forth in any one of SEQ ID NOs: 52-57, and the VL comprises a LCDR1, a LCDR2, and a LCDR3 from the amino acid sequence set forth in any one of SEQ ID NOs: 58-61,
wherein the CDRs are defined according to the Kabat, IMGT, Chothia, AbM, or Contact numbering scheme, for example, according to the Kabat numbering scheme.

In some specific embodiments, in the second antigen-binding domain,
the VH comprises a HCDR1, a HCDR2, and a HCDR3 of the amino acid sequences set forth in SEQ ID NOs: 42-44, and the VL comprises a LCDR1, a LCDR2, and a LCDR3 of the amino acid sequences set forth in SEQ ID NOs: 45-47;
the VH comprises a HCDR1, a HCDR2, and a HCDR3 of the amino acid sequences set forth in SEQ ID NOs: 50, 43, and 44, and the VL comprises a LCDR1, a LCDR2, and a LCDR3 of the amino acid sequences set forth in SEQ ID NOs: 45-47;
the VH comprises a HCDR1, a HCDR2, and a HCDR3 of the amino acid sequences set forth in SEQ ID NOs: 51, 43, and 44, and the VL comprises a LCDR1, a LCDR2, and a LCDR3 of the amino acid sequences set forth in SEQ ID NOs: 45-47.

In some specific embodiments, the second antigen-binding domain is engineered by humanization, back mutation, avidity maturation, T cell epitope (TCE) removal/decreasing the number of TCEs, reduction of antibody deamidation, and/or reduction of antibody isomerization.

In some specific embodiments, the second antigen-binding domain comprises mutations in the amino acid residues at positions 1, 30, 37, 44, 49, 73, 89, and/or 93 (numbering according to the Kabat numbering scheme) in the VH, and/or mutations in the amino acid residues at positions 4, 17, 36, 58, 60, and/or 68 (numbering according to the Kabat numbering scheme) in the VL, such as one, more, or any combination of mutations of 1E, 30T, 37V, 44G, 49G, 73N, 89R, and 93V in the VH, and/or one, more, or any combination of mutations of 4L, 17Q, 36F, 58I, 60A, and 68R in the VL.

In some specific embodiments, in the second antigen-binding domain,
the VH comprises the amino acid sequence set forth in SEQ ID NO: 40 or an amino acid sequence having at least 80% or at least 90% identity thereto, and the VL comprises the amino acid sequence set forth in SEQ ID NO: 41 or an amino acid sequence having at least 80% or at least 90% identity thereto;
the VH comprises the amino acid sequence set forth in any one of SEQ ID NOs: 52-57 or an amino acid sequence having at least 80% or at least 90% identity thereto, and the VL comprises the amino acid sequence set forth in any one of SEQ ID NOs: 58-61 or an amino acid sequence having at least 80% or at least 90% identity thereto;
for example, the VH comprises the amino acid sequence set forth in SEQ ID NO: 56, and the VL comprises the amino acid sequence set forth in SEQ ID NO: 60.

In some specific embodiments, the second antigen-binding domain comprises a heavy chain and a light chain, wherein the amino acid sequence of the heavy chain is set forth in SEQ ID NO: 62 or 64 or has at least 80% or at least 90% identity thereto, and the amino acid sequence of the light chain is set forth in SEQ ID NO: 63 or 65 or has at least 80% or at least 90% identity thereto.

In some specific embodiments, the second antigen-binding domain that specifically binds to IL-23 comprises a fab or a VH and VL in risankizumab and guselkumab. In some specific embodiments, the second antigen-binding domain that specifically binds to IL-23 comprises a VH and a VL, wherein the VH comprises a HCDR1, a HCDR2, and a HCDR3 from SEQ ID NO: 66, and the VL comprises a LCDR1, a LCDR2, and a LCDR3 from SEQ ID NO: 67; for example, the VH is the VH from SEQ ID NO: 66 or has at least 80% or at least 90% identity thereto, and the VL is the VL from SEQ ID NO: 67 or has at least 80% or at least 90% identity thereto. In some specific embodiments, the second antigen-binding domain that specifically binds to IL-23 comprises a VH and a VL, wherein the VH comprises a HCDR1, a HCDR2, and a HCDR3 from SEQ ID NO: 77, and the VL comprises a LCDR1, a LCDR2, and a LCDR3 from SEQ ID NO: 78; for example, the VH is the VH from SEQ ID NO: 77 or has at least 80% or at least 90% identity thereto, and the VL is the VL from SEQ ID NO: 78 or has at least 80% or at least 90% identity thereto.

### Regarding the IL-36R/IL-23-binding protein:

The IL-36R/IL-23-binding proteins of the present disclosure are provided below for illustrative purposes:
In some embodiments, the IL-36R/IL-23-binding protein comprises one or more (e.g., 2, 3, 4, 5, or 6) first antigen-binding domains that specifically bind to IL-36R and/or one or more (e.g., 2, 3, or 4) second antigen-binding domains that specifically bind to IL-23. In some specific embodiments, the IL-36R/IL-23-binding protein comprises 2 first antigen-binding domains that specifically bind to IL-36R and 2 second antigen-binding domains that specifically bind to IL-23.

In some embodiments, in the IL-36R/IL-23-binding protein, a valency ratio of the first antigen-binding domain that specifically binds to IL-36R to the second antigen-binding domain that specifically binds to IL-23 is between 6:1 and 1:3 (e.g., between 4:1 and 1:2), e.g., 1:1, 1:2, 2:1, 1:3, or 3:1.

In some embodiments, in the IL-36R1IL-23-binding protein, the first antigen-binding domain that specifically binds to IL-36R is located at the N-terminus and/or the C-terminus of the second antigen-binding domain that specifically binds to IL-23.

In some embodiments, the IL-36R/IL-23-binding protein further comprises an Fc region of a human immunoglobulin. For example, the Fc region is an Fc region of human IgG1, IgG2, or IgG4.

In some specific embodiments, the Fc region can cause the binding protein to form a dimeric molecule.

In some specific embodiments, the Fc region comprises a mutation that extends the *in vivo* half-life, which depends on the FcRn binding avidity. The extension of half-life can allow for a decrease in the amount of a drug given to a patient and/or a reduction in the frequency of administration. For example, the Fc region comprises an M252Y, S254T, and/or T256E mutation.

In some specific embodiments, the Fc region may be an Fc region with a reduced effector function; for example, the Fc region may comprise a mutation, and exemplary IgG Fc regions with a reduced effector function comprise the following substitutions: N297A or N297Q (IgGl); L234A/L235A (IgG1); V234A/G237A (IgG2); L235A/G237A/E318A (IgG4); H268Q/V309L/A330S/A331S (IgG2); C220S/C226S/C229S/P238S (IgG1); C226S/C229S/E233P/L234V/L235A (IgG1); L234F/L235E/P331S (IgG1); or S267E/L328F (IgG1).

In some embodiments, the Fc region is an Fc region with increased stability, such as an Fc region of human IgG4 comprising an S228P mutation.

In some embodiments, the amino acid sequence of the Fc region is set forth in SEQ ID NO: 11, 48, or 76 or has at least 80% or at least 90% identity thereto.

In some embodiments, the Fc region in the IL-36R/IL-23-binding protein comprises a first subunit Fc1 and a second subunit Fc2 capable of associating with each other.

In some embodiments, Fc1 and Fc2 comprise such amino acid mutations that Fc1 preferentially pairs with Fc2 or preferentially forms a heterodimer as compared to Fc1. In some embodiments, the mutations are located in the CH3 domains of Fc1 and Fc2. In some embodiments, the amino acid mutations in Fc1 and Fc2 result in greater electrostatic complementarity than a wild type without the mutations. In some embodiments, the amino acid mutations in Fc1 and Fc2 result in greater steric complementarity than a wild type without the mutations.

In some embodiments, in Fc1 and Fc2, for example, within the CH3/CH3 interface, one or more amino acid residues in the CH3 domain of Fc1 are mutated with one or more amino acid residues having a larger side-chain volume, thereby forming protuberances (or knobs) in the surface of the CH3 domain of Fc1; one or more, preferably two or three, of the amino acid residues in the CH3 domain of Fc2 that interact with the CH3 domain of Fc1 are mutated with amino acid residues having a smaller side-chain volume, thereby forming cavities (or holes) in the surface of the CH3 domain of Fc2 that interacts with the CH3 domain of Fc1. In some embodiments, an import residue having a larger side-chain volume is phenylalanine (F), tyrosine (Y), arginine (R), or tryptophan (W). In some embodiments, an import residue having a smaller side-chain volume is serine (S), alanine (A), valine (V), or threonine (T).

In some specific embodiments, the Fc1 comprises at least one or at least two amino acid mutations selected from the group consisting of T366S, L368A, and Y407V (hole mutation modifications), and the Fc2 comprises T366W (a knob mutation modification); or the Fc1 comprises T366W (a knob mutation modification), and the Fc2 comprises at least one or at least two amino acid mutations selected from the group consisting of T366S, L368A, and Y407V (hole mutation modifications).

In some specific embodiments, Fc1 and Fc2 may comprise natural-non-cysteine-to-cysteine mutations, for example, in the CH3 domains; for example, Fc1 comprises S354C, and Fc2 comprises Y349C; or Fc1 comprises Y349C, and Fc2 comprises S354C.

In some specific embodiments, Fc1 and Fc2, for example, within the Fc1 CH3/Fc2 CH3 interface, comprise the following amino acid mutations or a combination thereof: T366Y/Y407T; T366W/Y407A; T366Y/Y407T; T394W/F405A; T366Y/F405AT394W/ Y407T; T366W/F405WT394S/Y407A; F405W/T394S; D399C/K392C; T366W/T366S/ L368A/Y407V; T366W/D399C/T366S/L368A/K392C/Y407V; T366W/K392C/T366S/ D399C/L368A/Y407V; S354C/T366W/Y349C/T366S/L368A/Y407V; Y349C/T366W/ S354C/T366S/L368A/Y407V; E356C/T366W/Y349C/T366S/L368A/Y407V; Y349C/ T366W/E356C/T366S/L368A/Y407V; E357C/T366W/Y349C/T366S/L368A/Y407V; and Y349C/T366W/E357C/T366S/L368A/Y407V.

In some specific embodiments, Fc1 and Fc2 further comprise amino acid mutations that cause the formation of an electrostatic interaction interface between Fc1 and Fc2 (e.g., CH3 and CH3). The amino acid mutations that cause the formation of the electrostatic interaction interface are selected from the group consisting of, for example, K370E/D399K/K439D/D356K/E357K/K409D; K409D/D399K; K409E/D399K; K409E/D399R; K409D/D399R; D339K/E356K; D399K/E356K/K409D/K392D; D399K/E356K/K409D/K439D; D399K/E357K/K409D/K370D; D399K/E356K/E357K/ K409D/K392D/K370D; D399K/E357K/K409D/K392D; K392D/K409D/D399K; and K409D/K360D/D399K.

In some embodiments, Fc1 and/or Fc2 comprise(s) domains from different antibody subtypes, e.g., CH3 domains from different antibody subtypes.

Additionally, the present disclosure cites the scheme of modifying the CH3 region of the Fc region to enhance heterodimerization in WO96/27011, WO98/050431, EP1870459, WO2007/110205, WO2007/147901, WO2009/089004, WO2010/129304, WO2011/ 90754, WO2011/143545, WO2012058768, WO2013157954, and WO2013096291.

In some embodiments, in the IL-36R/IL-23-binding protein, the first antigen-binding domain that specifically binds to IL-36R and the second antigen-binding domain that specifically binds to IL-23 are linked, either directly or by a linker. In some embodiments, the second antigen-binding domain that specifically binds to IL-23 and the Fc region are linked, either directly or by a linker.

In some specific embodiments, the linker includes, but is not limited to, amino acid sequences represented by (GₘSₙ)ₕ or (GGNGT)ₕ or (YGNGT)ₕ or (EPKSS)ₕ, wherein m and n are each independently selected from the group consisting of an integer of 1-8 (e.g., 1, 2, 3, 4, 5, 6, 7, or 8), and h is independently selected from the group consisting of an integer of 1-20 (e.g., 1, 2, 3, 4, 5, 6, 7, 8, 9, 10, 11, 12, 13, 14, 15, 16, 17, 18, 19, or 20). In some specific embodiments, the linker may be a non-functional amino acid sequence of 1-20 or more amino acids without a secondary or higher structure. In some specific embodiments, the linker is a flexible linker. In some specific embodiments, the linker is selected from the group consisting of G₄S, GS, GAP, (G₄S)₂, (G₄S)₃, (G₄S)₄, (G₄S)₅, and ASGS; for example, the linker is (G₄S)₂ or (G₄S)₃.

In some specific embodiments, the IL-36R/IL-23-binding protein comprises a first polypeptide chain and a second polypeptide chain, wherein the first and second polypeptide chains are as follows, from the N-terminus to the C-terminus:
(1) the first polypeptide chain: [the first antigen-binding domain that specifically binds to IL-36R]-[linker 1]a-[the VH of the second antigen-binding domain that specifically binds to IL-23]-CH1-Fc region; the second polypeptide chain: [the VL of the second antigen-binding domain that specifically binds to IL-23]-CL;
(2) the first polypeptide chain: [the VH of the second antigen-binding domain that specifically binds to IL-23]-CH1-Fc region; the second polypeptide chain: [the first antigen-binding domain that specifically binds to IL-36R]-[linker 1]a-[the VL of the second antigen-binding domain that specifically binds to IL-23]-CL;
(3) the first polypeptide chain: [the VH of the second antigen-binding domain that specifically binds to IL-23]-CH1-Fc region-[linker 1]a-[the first antigen-binding domain that specifically binds to IL-36R]; the second polypeptide chain: [the VL of the second antigen-binding domain that specifically binds to IL-23]-CL;
(4) the first polypeptide chain: [the VH of the second antigen-binding domain that specifically binds to IL-23]-CH1-Fc region; the second polypeptide chain: [the VL of the second antigen-binding domain that specifically binds to IL-23]-[linker 1]a-CL[the first antigen-binding domain that specifically binds to IL-36R];
(5) the first polypeptide chain: [the VH of the second antigen-binding domain that specifically binds to IL-23]-CH1-Fc region-[linker 1]a-[the first antigen-binding domain that specifically binds to IL-36R]; the second polypeptide chain: [the VL of the second antigen-binding domain that specifically binds to IL-23]-CL-[linker 2]b-[the first antigen-binding domain that specifically binds to IL-36R];
(6) the first polypeptide chain: [the first antigen-binding domain that specifically binds to IL-36R]-[linker 1]a-[the VH of the second antigen-binding domain that specifically binds to IL-23]-CH1-Fc region; the second polypeptide chain: [the first antigen-binding domain that specifically binds to IL-36R]-[linker 2]b-[the VL of the second antigen-binding domain that specifically binds to IL-23]-CL,
wherein - represents a peptide bond; the linkers are polypeptides capable of fulfilling the function of linking; linker 1 and linker 2 may be identical or different; a and b may be independently selected from the group consisting of 1 and 0; for example, a and b are both 1. The linkers are, for example, selected from the group consisting of G₄S, GS, GAP, (G₄S)₂, (G₄S)₃, (G₄S)₄, (G₄S)₅, and ASGS; for example, the linkers are (G₄S)₂ or (G₄S)₃. In some embodiments, provided is an IL-36R/IL-23-binding protein comprising a first polypeptide chain and a second polypeptide chain. In some specific embodiments, the amino acid sequence of the first polypeptide chain is set forth in any one of SEQ ID NOs: 68, 70, 71, 74, and 75 or has at least 80% or at least 90% sequence identity thereto, and the amino acid sequence of the second polypeptide chain is set forth in any one of SEQ ID NOs: 69, 72, and 73 or has at least 80% or at least 90% sequence identity thereto.

In some embodiments, provided is an IL-36R/IL-23-binding protein comprising a first polypeptide chain and a second polypeptide chain:
(1) the amino acid sequence of the first polypeptide chain is set forth in SEQ ID NO: 68 or 70 or has at least 80% or at least 90% sequence identity thereto, and the amino acid sequence of the second polypeptide chain is set forth in SEQ ID NO: 69 or has at least 80% or at least 90% sequence identity thereto;
(2) the amino acid sequence of the first polypeptide chain is set forth in SEQ ID NO: 71 or has at least 80% or at least 90% sequence identity thereto, and the amino acid sequence of the second polypeptide chain is set forth in SEQ ID NO: 72 or 73 or has at least 80% or at least 90% sequence identity thereto;
(3) the amino acid sequence of the first polypeptide chain is set forth in SEQ ID NO: 74 or 75 or has at least 80% or at least 90% sequence identity thereto, and the amino acid sequence of the second polypeptide chain is set forth in SEQ ID NO: 69 or has at least 80% or at least 90% sequence identity thereto.

In some embodiments, the IL-36R/IL-23-binding protein of the present disclosure has an activity selected from the group consisting of at least one of the following:
(a) binding to human IL-36R or an epitope thereof with a K_{D} value of ≤10⁻⁷;
(b) binding to a human IL-23 p19 subunit or an epitope thereof with a K_{D} value of ≤10⁻⁷;
(c) having the activity of blocking IL-23/IL-23R binding; preferably, blocking human IL-23/IL-23R binding with an IC50 value of less than 0.6 nM (e.g., less than 0.6 nM, less than 0.5 nM, less than 0.4 nM, less than 0.3 nM, or less), the IC₅₀ value being measured by ELISA, wherein in some embodiments, the IC₅₀ value is measured by the method in Example 5 of the present disclosure;
(d) having the activity of inhibiting IL-17 secretion; preferably, inhibiting IL-17 secretion with an IC₅₀ value of less than 0.03 nM (e.g., less than 0.03 nM, less than 0.02 nM, less than 0.01 nM, or less), wherein in some embodiments, the IC₅₀ value is measured by the method in Example 5 of the present disclosure;
(e) having the activity of inhibiting proliferation of BaF3-IL-23R cells; preferably, inhibiting proliferation of BaF3-IL-23R cells with an IC₅₀ value of less than 0.5 nM (e.g., less than 0.5 nM, less than 0.4 nM, less than 0.3 nM, less than 0.2 nM, less than 0.1 nM, or less), wherein in some embodiments, the IC₅₀ value is measured by the method in Example 9;
(f) having the activity of inhibiting the STAT pathway; preferably, inhibiting the STAT3 pathway with an IC₅₀ value of less than 10 nM (e.g., less than 9 nM, less than 8 nM, or less), wherein in some embodiments, the IC₅₀ value is measured by the method in Example 9;
(g) having the activity of inhibiting a human IL-36 stimulating factor; preferably, inhibiting IL-8 secretion with an IC₅₀ value of less than 0.5 nM (e.g., less than 0.5 nM, less than 0.4 nM, less than 0.3 nM, less than 0.2 nM, less than 0.1 nM, or less), wherein in some embodiments, the IC₅₀ value is measured by the method in Example 8;
(h) capable of alleviating or treating an inflammatory disease caused by IL-36 and/or IL-23 overexpression, e.g., psoriasis; preferably, capable of inhibiting human IL-36- and human IL-23-induced mouse ear swelling in a mouse animal model, with an inhibition rate of, e.g., at least about 10%, e.g., at least about 20%, about 30%, about 40%, about 50%, about 60%, about 70%, about 80%, about 90%, or more, wherein in some embodiments, the inhibition ratio is determined by measuring IL-36α, IL-23, IL-17F, S100A8, S100A9, and KRT16, e.g., by the method in Example 10.

In some embodiments, the IL-36R/IL-23-binding protein of the present disclosure encompasses a variant, wherein the variant comprises one or more (e.g., 1, 2, 3, 4, 5, 6, 7, 8, 9, or 10) amino acid mutations as compared to the first and second polypeptide chains in any one of the aforementioned combinations (1)-(3); the amino acid mutations may be conservative replacements, substitutions, or modifications, and/or deletions or additions that do not affect function.

In some embodiments, provided is an IL-36R/IL-23-binding protein that binds to or competes with the aforementioned IL-36R/IL-23-binding protein of the present disclosure for binding to IL-23 and/or IL-36R, or the same epitope of IL-23 and/or IL-36R.

In some embodiments, provided is an IL-36R/IL-23-binding protein that blocks the binding of the aforementioned IL-36R/IL-23-binding protein of the present disclosure to IL-23 and/or IL-36R.

In some embodiments, provided is a protein or molecule comprising any of the aforementioned IL-36R/IL-23-binding proteins of the present disclosure. For example, the protein or molecule is a conjugate, and the conjugate may, for example, comprise any detectable label.

### Composition or Complex of IL-36R-Binding Protein and IL-23-Binding Protein

The present disclosure provides a composition or complex comprising an IL-36R-binding protein and an IL-23-binding protein, e.g., an anti-IL-36R antibody or an antigen-binding fragment thereof and an anti-IL-23 antibody or an antigen-binding fragment thereof.

In some embodiments, the IL-36R-binding protein is the aforementioned IL-36R-binding protein of the present disclosure, and the IL-23-binding protein comprises a VH and a VL, wherein the VH comprises a HCDR1, a HCDR2, and a HCDR3 from the amino acid sequence set forth in SEQ ID NO: 40, and the VL comprises a LCDR1, a LCDR2, and a LCDR3 from the amino acid sequence set forth in SEQ ID NO: 41; or the VH comprises a HCDR1, a HCDR2, and a HCDR3 from the amino acid sequence set forth in any one of SEQ ID NOs: 52-57, and the VL comprises a LCDR1, a LCDR2, and a LCDR3 from the amino acid sequence set forth in any one of SEQ ID NOs: 58-61, wherein the CDRs are defined according to the Kabat, IMGT, Chothia, AbM, or Contact numbering scheme, for example, according to the Kabat numbering scheme.

In some specific embodiments, in the IL-23-binding protein,
the VH comprises a HCDR1, a HCDR2, and a HCDR3 of the amino acid sequences set forth in SEQ ID NOs: 42-44, and the VL comprises a LCDR1, a LCDR2, and a LCDR3 of the amino acid sequences set forth in SEQ ID NOs: 45-47;
the VH comprises a HCDR1, a HCDR2, and a HCDR3 of the amino acid sequences set forth in SEQ ID NOs: 50, 43, and 44, and the VL comprises a LCDR1, a LCDR2, and a LCDR3 of the amino acid sequences set forth in SEQ ID NOs: 45-47;
the VH comprises a HCDR1, a HCDR2, and a HCDR3 of the amino acid sequences set forth in SEQ ID NOs: 51, 43, and 44, and the VL comprises a LCDR1, a LCDR2, and a LCDR3 of the amino acid sequences set forth in SEQ ID NOs: 45-47.

In some specific embodiments, in the IL-23-binding protein,
the VH comprises the amino acid sequence set forth in SEQ ID NO: 40 or an amino acid sequence having at least 80% or at least 90% identity thereto, and the VL comprises the amino acid sequence set forth in SEQ ID NO: 41 or an amino acid sequence having at least 80% or at least 90% identity thereto;
the VH comprises the amino acid sequence set forth in any one of SEQ ID NOs: 52-57 or an amino acid sequence having at least 80% or at least 90% identity thereto, and the VL comprises the amino acid sequence set forth in any one of SEQ ID NOs: 58-61 or an amino acid sequence having at least 80% or at least 90% identity thereto.

Illustratively, provided is a composition or complex comprising an IL-36R-binding protein and an IL-23-binding protein, wherein the IL-36R-binding protein comprises or is VHH 213 or a humanized and back-mutated sequence thereof (e.g., VHH213_Hu07) in Examples 1-3 of the present disclosure, or is VHH162 or a humanized and back-mutated sequence thereof (e.g., VHH162_Hu11); the IL-23-binding protein comprises or is the anti-IL-23 antibody (e.g., Hu29-19) in Example 4 of the present disclosure.

### Polynucleotide and Vector

The present disclosure provides a polynucleotide encoding the IL-36R-binding protein or the IL-36R/IL-23-binding protein of the present disclosure. The nucleic acid of the present disclosure may be an RNA, DNA, or cDNA. According to some embodiments of the present disclosure, the nucleic acid of the present disclosure is a substantially isolated nucleic acid.

The nucleic acid of the present disclosure may also be in the form of a vector and may be present in the vector and/or may be part of the vector. The vector is, for example, a plasmid, cosmid, YAC, or viral vector. The vector may especially be an expression vector, i.e., a vector that provides for *in vitro* and/or *in vivo* (i.e., in suitable host cells, host organisms, and/or expression systems) expression of the IL-36R-binding protein or the IL-36R/IL-23-binding protein. The expression vector generally comprises at least one of the nucleic acids of the present disclosure, which is operably linked to one or more suitable expression regulatory elements (e.g., promoters, enhancers, and terminators). The selection of the elements and sequences thereof for expression in a particular host is common knowledge of those skilled in the art. Regulatory elements and other elements useful or necessary for expression of the IL-36R-binding protein and the IL-36R/IL-23-binding protein of the present disclosure include, for example, promoters, enhancers, terminators, integration factors, selection labels, leader sequences, and reporter genes.

The nucleic acid of the present disclosure may be prepared or obtained by known means (e.g., by automatic DNA synthesis and/or recombinant DNA techniques) based on information on the amino acid sequence of the polypeptide of the present disclosure, and/or may be isolated from a suitable natural source.

### Host Cell

The present disclosure provides a recombinant host cell that expresses or is capable of expressing one or more of the IL-36R-binding proteins and IL-36R/IL-23-binding proteins of the present disclosure and/or comprises the polynucleotide or vector of the present disclosure. In some embodiments, the host cell is a bacterial cell, a fungal cell, or a mammalian cell.

Bacterial cells include, e.g., cells of gram-negative bacterial strains (e.g., *Escherichia coli* strains, *Proteus* strains, and *Pseudomonas* strains), and gram-positive bacterial strains (e.g., *Bacillus* strains, *Streptomyces* strains, *Staphylococcus* strains, and *Lactococcus* strains).

Fungal cells include, e.g., cells of the species *Trichoderma, Neurospora,* and *Aspergillus;* or cells of the species *Saccharomyces* (e.g., *Saccharomyces cerevisiae*), *Schizosaccharomyces* (e.g., *Schizosaccharomyces pombe*), *Pichia* (e.g., *Pichia pastoris* and *Pichia methanolica*), and *Hansenula.*

Mammalian cells include, e.g., HEK293 cells, CHO cells, BHK cells, HeLa cells, COS cells, and the like.

However, amphibian cells, insect cells, plant cells, and any other cells used in the art for expressing heterologous proteins may also be used in the present disclosure.

### Preparation Method

The present disclosure provides a method for preparing an IL-36R-binding protein or an IL-36R1IL-23-binding protein, the method comprising: expressing the target protein in the aforementioned host cell and isolating the target protein from the host cell. Optionally, the method may further comprise a purification step, for example, purifying using an A or G Sepharose FF column containing an adjusted buffer to wash off non-specifically bound components, then eluting bound antibodies using a pH gradient method, detecting using SDS-PAGE, and collecting. Optionally, filtration and concentration are performed by conventional methods. Soluble mixtures and multimers can also be removed by conventional methods, such as molecular sieves and ion exchange. The resulting products need to be immediately frozen, such as at -70 °C, or lyophilized.

Methods for producing and purifying antibodies are well known in the prior art and can be found in, for example, "Antibodies: A Laboratory Manual", Cold Spring Harbor Press (chapters 5-8 and 15).

The engineered antibodies or antigen-binding fragments of the present disclosure can be prepared and purified by conventional methods. For example, cDNA sequences encoding heavy and light chains can be cloned and recombined into an expression vector. CHO cells can be stably transfected with recombinant immunoglobulin expression vectors. Mammalian expression systems may result in glycosylation of antibodies, particularly at the highly conserved N-terminus of the Fc region. Stable clones are obtained by expression of antibodies specifically binding to human antigens. Positive clones are expanded in a serum-free culture medium in a bioreactor to produce antibodies. The culture solution with the secreted antibodies can be purified and collected by conventional techniques. The antibodies can be filtered and concentrated by conventional methods. Soluble mixtures and multimers can also be removed by conventional methods, such as molecular sieves and ion exchange.

### Composition

The present disclosure provides a composition comprising the aforementioned IL-36R-binding protein or IL-36R/IL-23-binding protein of the present disclosure. For example, provided is a pharmaceutical composition comprising an amount of the aforementioned IL-36R-binding protein or IL-36R/IL-23-binding protein that is effective in treating, alleviating, or preventing a disease, and at least one pharmaceutically acceptable excipient, diluent, or carrier.

In some specific embodiments, a unit dose of the pharmaceutical composition may comprise 0.01 to 99 weight% of the IL-36R-binding protein or the IL-36R1IL-23-binding protein, or a unit dose of the pharmaceutical composition comprises 0.1-2000 mg of the IL-36R-binding protein or the IL-36R/IL-23-binding protein. In some specific embodiments, a unit dose of the pharmaceutical composition comprises 1-1000 mg of the IL-36R-binding protein or the IL-36R/IL-23-binding protein.

In some embodiments, provided is an article of manufacture or product comprising the aforementioned IL-36R-binding protein or IL-36R/IL-23-binding protein. Optionally, the article of manufacture comprises a container and a label. Examples of containers are bottles, syringes, and test tubes. The container accommodates a composition effective in treating a disorder. The label on or associated with the container indicates that the composition is used for treating the disorder of choice.

In some embodiments, the aforementioned disease is an inflammatory disease or an autoimmune disease.

### Treatment Method and Pharmaceutical Use

The present disclosure provides the aforementioned IL-36R-binding protein, IL-36R/IL-23-binding protein, polynucleotide, or composition (including the pharmaceutical composition) for use in treating, alleviating, preventing, or diagnosing a disease or disorder.

In some embodiments, provided is a method for ameliorating, alleviating, treating, or preventing a disease, the method comprising administering to a subject an amount of the aforementioned IL-36R-binding protein, IL-36R/IL-23-binding protein, polynucleotide, or composition (including the pharmaceutical composition) that is effective in ameliorating, alleviating, treating, or preventing the disease.

In some embodiments, provided is use of the IL-36R-binding protein, the IL-36R/IL-23-binding protein, the polynucleotide, or the composition (including the pharmaceutical composition) of the present disclosure in the manufacture of a medicament for ameliorating, alleviating, treating, or preventing a disease.

In some embodiments, the aforementioned disease is a disease or disorder associated with IL-36 and/or IL-23 overexpression.

In some embodiments, the aforementioned disease is an IL-36R-mediated disease or disorder.

In some embodiments, the aforementioned disease is an inflammatory disease or an autoimmune disease, such as psoriasis.

### Detection

The present disclosure provides use of the IL-36R-binding protein, the IL-36R/IL-23-binding protein, the polynucleotide, or the composition for detection. The present disclosure further provides a method, system, or device for detecting IL-36R or IL-23 *in vivo* or *in vitro,* comprising treating a sample with the aforementioned binding protein, polynucleotide, or composition of the present disclosure.

In some embodiments, the method, system, or device for *in vitro* detection may comprise, for example,
(1) contacting the sample with the IL-36R-binding protein, the IL-36R/IL-23-binding protein, the polynucleotide, or the composition of the present disclosure;
(2) detecting a complex formed between the aforementioned binding protein or polynucleotide and the sample; and/or
(3) contacting a reference sample (e.g., a control sample) with the binding protein or nucleic acid; and
(4) determining the extent of formation of the complex by comparison with the reference sample. A change (e.g., a statistically significant change) in complex formation in the sample as compared to the control sample indicates the presence of IL-36R or IL-23 in the sample.

In some embodiments, further provided is a kit comprising the aforementioned IL-36R-binding protein, IL-36R/IL-23-binding protein, or polynucleotide, wherein the kit may further comprise instructions for diagnostic use. The kit may further comprise at least one additional reagent, such as a label or an additional diagnostic agent. For *in vivo* use, the IL-36R-binding protein or the IL-36R/IL-23-binding protein may be formulated into a pharmaceutical composition.

### Definitions of Terms

In order to facilitate the understanding of the present disclosure, certain technical and scientific terms are specifically defined below. Unless otherwise specifically defined in the present disclosure, all other technical and scientific terms used in the present disclosure have the meanings generally understood by those of ordinary skill in the art to which the present disclosure pertains.

The three-letter and single-letter codes for amino acids used in the present disclosure are as described in J. biol. chem., 243, p3558 (1968).

"IL-23" (also known as IL23) is mainly produced by activated dendritic cells, macrophages, monocytes, etc. It is a member of the IL-12 heterodimer cytokine family and consists of two subunits, p19 (also known as IL-23 p19 subunit) and p40 (also known as IL-23 p40 subunit), wherein the p40 subunit is a subunit also contained in IL-12 *(*J Immunol. 2018 Sep 15; 201(6): 1605-1613).

"IL-36R-binding protein" encompasses any protein capable of specifically binding to IL-36R or any molecule comprising the protein, including but not limited to, antibodies targeting IL-36R as defined in the present disclosure, antigen-binding fragments thereof or conjugates thereof, and fusion proteins. In some embodiments, the "IL-36R-binding protein" may comprise at least one (e.g., 1, 2, 3, 4, 5, 6, or more) single-domain antibody that specifically binds to IL-36R in the examples of the present disclosure. In some embodiments, the "IL-36R-binding protein" of the present disclosure may comprise, in addition to the immunoglobulin single variable domain of IL-36R, a linker and/or a moiety with effector functions, such as a half-life extending moiety (e.g., an immunoglobulin single variable domain that binds to serum albumin) and/or a fusion partner (e.g., serum albumin) and/or a conjugated polymer (e.g., PEG) and/or an Fc region. In some embodiments, the "IL-36R/IL-23-binding protein" encompasses the anti-IL-36R/IL-23 bispecific antibodies in the examples of the present disclosure.

"IL-36R/IL-23-binding protein" encompasses any protein capable of specifically binding to IL-23 and IL-36R or any molecule comprising the protein, including but not limited to, antibodies, polypeptides, fusion proteins of antibodies and polypeptides, or conjugates thereof, and fusion proteins. In some embodiments, the "IL-36R/IL-23-binding protein" encompasses the anti-IL-36R/IL-23 bispecific antibodies in the examples of the present disclosure.

"Antibody" encompasses a variety of antibody structures, including but not limited to, monoclonal antibodies, polyclonal antibodies; monospecific antibodies, multispecific antibodies (e.g., bispecific antibodies); and full-length antibodies, and antibody fragments (or antigen-binding fragments, or antigen-binding moieties) as long as they exhibit the desired antigen-binding activity. An antibody may refer to an immunoglobulin that is a four-peptide-chain structure formed by linking two identical heavy chains and two identical light chains by interchain disulfide bonds. The heavy chain constant regions of immunoglobulins differ in their amino acid composition and arrangement, and thus in their antigenicity. Accordingly, immunoglobulins can be divided into five classes, or isotypes of immunoglobulins, namely IgM, IgD, IgG, IgA, and IgE, with their corresponding heavy chains being µ chain, δ chain, γ chain, α chain, and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3, and IgG4. Light chains are divided into κ or λ chains according to differences in the constant regions. Each of the five classes of Ig may have a κ chain or λ chain. In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (HVRs) and 4 framework regions (FRs) with relatively conservative sequences. The 3 hypervariable regions determine the specificity of the antibody and thus are also known as complementarity-determining regions (CDRs). Each of the light chain variable regions (VLs) and the heavy chain variable regions (VHs) consists of 3 CDRs and 4 FRs arranged from the amino-terminus to the carboxyl-terminus as follows: FR1, CDR1, FR2, CDR2, FR3, CDR3, and FR4. The 3 CDRs of the light chain refer to LCDR1, LCDR2, and LCDR3; and the 3 CDRs of the heavy chain refer to HCDR1, HCDR2, and HCDR3.

The antibodies of the present disclosure may be polyclonal, monoclonal, xenogenic, allogeneic, syngeneic, or modified forms thereof, with the monoclonal antibodies being especially applicable in various examples. Generally, the antibodies of the present disclosure are recombinant antibodies. As used herein, "recombinant" generally refers to such products as a cell, a nucleic acid, a protein, or a vector, and indicates that the cell, the nucleic acid, the protein, or the vector has been modified by introduction of a heterologous nucleic acid or protein or alteration of a natural nucleic acid or protein, or that the cell is derived from a cell modified in this way. For example, recombinant cells express genes that are not found within the native (non-recombinant) cellular form or express native genes that are abnormally expressed, lowly expressed, or not expressed at all.

"Antigen-binding fragment" encompasses a single-chain antibody (i.e., full-length heavy and light chains); a Fab, a modified Fab, a Fab', a modified Fab', a F(ab')2, an Fv, a Fab-Fv, a Fab-dsFv, a single-domain antibody (e.g., VH or VL or VHH), an scFv, a bivalent or trivalent or tetravalent antibody, a Bis-scFv, a diabody, a tribody, a triabody, a tetrabody, and an epitope-binding fragment of any one of the above (see, e.g., Holliger and Hudson, 2005, Nature Biotech. 23(9):1126-1136; Adair and Lawson, 2005, Drug Design Reviews-Online 2(3), 209-217). Methods for producing and preparing such antigen-binding fragments are well known in the art (see, e.g., Verma et al., 1998, Journal of Immunological Methods, 216, 165-181).

For the determination or definition of CDRs, the deterministic depiction of CDRs and identification of residues comprising antigen-binding sites of an antibody can be accomplished by resolving the structure of the antibody and/or resolving the structure of the antibody-ligand complex. This can be accomplished by any one of a variety of techniques known to those skilled in the art, such as X-ray crystallography. A variety of analysis methods can be used to identify CDRs, including but not limited to the Kabat numbering scheme, the Chothia numbering scheme, the AbM numbering scheme, the IMGT numbering scheme, the contact definition, and the conformational definition. The Kabat numbering scheme is a standard for numbering residues in antibodies and is generally used to identify CDRs (see, e.g., Johnson & Wu, 2000, Nucleic Acids Res., 28:214-8). The Chothia numbering scheme is similar to the Kabat numbering scheme, except that it takes into account the position of certain structural loop regions (see, e.g., Chothia et al., 1986, J. Mol. Biol., 196:901-17; Chothia et al., 1989, Nature, 342:877-83). The AbM numbering scheme adopts a computer program integration suite for modeling antibody structures manufactured by Oxford Molecular Group (see, e.g., Martin et al., 1989, Proc Natl Acad Sci (USA), 86:9268-9272; "AbMTM, A Computer Program for Modeling Variable Regions of Antibodies", Oxford, UK; Oxford Molecular, Ltd.). The AbM numbering scheme adopts a combination of a knowledge database and the de-novo method to model the tertiary structure of antibodies from basic sequences (see those described in Samudrala et al., 1999, "Ab Initio Protein Structure Prediction Using a Combined Hierarchical Approach", PROTEINS, Structure, Function and Genetics Suppl., 3:194-198). The contact definition is based on the analysis of the available complex crystal structures (see, e.g., MacCallum et al., 1996, J. Mol. Biol., 5:732-45). In the conformational definition, the positions of the CDRs can be identified as residues that contribute enthalpy to the antigen binding (see, e.g., Makabe et al., 2008, Journal of Biological Chemistry, 283:1156-1166). In addition, other CDR boundary definitions may not strictly follow one of the methods described above, but still overlap with at least a portion of the Kabat CDRs, although they may be shortened or lengthened based on predictions or experimental results that a particular residue or a particular group of residues do not significantly affect the antigen binding. As used in the present disclosure, a CDR may refer to a CDR defined by any method known in the art, including combinations of methods. The correspondence between the various numbering schemes is well known to those skilled in the art.

"Domain" of a polypeptide or protein refers to a folded protein structure that is capable of maintaining its tertiary structure independently of the rest of the protein. In general, a domain is responsible for a single functional property of a protein, and in many cases may be added, deleted, or transferred to other proteins without loss of functions of the rest of the protein and/or the domain.

"Immunoglobulin domain" refers to a globular region of an antibody chain (e.g., a chain of a conventional antibody with a four-peptide-chain structure or a heavy-chain antibody) or a polypeptide essentially consisting of such globular regions. The immunoglobulin domain is characterized in that it retains the immunoglobulin fold characteristic of an antibody molecule, and it consists of a 2-layer sandwich of about 7 antiparallel β-strands arranged in two β-sheets, optionally stabilized by a conserved disulfide bond.

"Immunoglobulin variable domain" refers to an immunoglobulin domain essentially consisting of four "framework regions" referred to in the art and hereinafter as "framework region 1" or "FR1", "framework region 2" or "FR2", "framework region 3" or "FR3", and "framework region 4" or "FR4", wherein the framework regions are interrupted by three "complementarity-determining regions" or "CDRs" referred to in the art and hereinafter as "complementarity-determining region 1" or "CDR1", "complementarity-determining region 2" or "CDR2", and "complementarity-determining region 3" or "CDR3". Thus, the general structure or sequence of an immunoglobulin variable domain can be expressed as follows: FR1-CDR1-FR2-CDR2-FR3-CDR3-FR4. Immunoglobulin variable domains possess specificity for an antigen by virtue of having an antigen-binding site.

"Antibody framework (FR)" refers to a portion of a variable domain, which serves as a framework for the antigen-binding loops (CDRs) of the variable domain.

"Immunoglobulin single variable domain" is generally used to refer to an immunoglobulin variable domain (which may be a heavy or light chain domain, including a VH, VHH, or VL domain) that can form a functional antigen-binding site without interacting with other variable domains (e.g., without VH/VL interactions as are required between the VH and VL domains of conventional four-chain monoclonal antibodies). Examples of "immunoglobulin single variable domains" include nanobodies (including VHHs, humanized VHHs, and/or camelized VHs, e.g., camelized human VHs), IgNARs, domains, (single-domain) antibodies as VH domains or derived from VH domains (such as dAbs^{™}) and (single-domain) antibodies as VL domains or derived from VL domains (such as dAbs^{™}). Immunoglobulin single variable domains based on and/or derived from heavy chain variable domains (such as VH or VHH domains) are generally preferred. A specific example of an immunoglobulin single variable domain is a "VHH domain" (or "VHH" for short) as defined below.

"VHH", also known as a heavy-chain single-domain antibody, VHH, V_{H}H domain, VHH antibody fragment, VHH antibody, or nanobody, is a variable domain of an antigen-binding immunoglobulin known as a "heavy-chain antibody" (i.e., "an antibody devoid of light chains") (Hamers-Casterman C, Atarhouch T, Muyldermans S, Robinson G, Hamers C, Songa EB, Bendahman N, Hamers R., "Naturally occurring antibodies devoid of light chains"; Nature 363, 446-448 (1993)). "VHH" is used to distinguish the variable domain from the heavy chain variable domain (which is referred to in the present disclosure as a "VH domain" or VH) and the light chain variable domain (which is referred to in the present disclosure as a "VL domain" or VL) present in conventional antibodies with a four-peptide-chain structure. VHH domains specifically bind to an epitope without the need for an additional antigen-binding domain (as opposed to the VH or VL domain in conventional antibodies with a four-peptide-chain structure, in which case the epitope is recognized by the VL domain together with the VH domain). A VHH domain is a small, stable, and efficient antigen recognition unit formed by a single immunoglobulin domain. The terms "heavy-chain single-domain antibody", "VHH domain", "VHH", "V_{H}H domain", "VHH antibody fragment", "VHH antibody", "Nanobody^{®}", and "Nanobody^{®} domain" ("Nanobody" is a trademark of Ablynx N.V., Ghent, Belgium) are used interchangeably. VHHs include, but are not limited to, natural antibodies produced by camelids, antibodies produced by camelids and then humanized, or antibodies obtained by screening using phage display techniques. The total number of amino acid residues in a VHH will usually be in the range of 110 to 120, often between 112 and 115. However, it should be noted that smaller and longer sequences may also be suitable for the purposes described in the present disclosure. Methods for obtaining VHHs that bind to a particular antigen or epitope have been previously disclosed in the following documents: R. van der Linden et al., Journal of Immunological Methods, 240 (2000) 185-195; Li et al., J Biol Chem., 287 (2012) 13713-13721; Deffar et al., African Journal of Biotechnology Vol. 8 (12), pp.2645-2652, 17 June, 2009, and WO94/04678.

As is well known in the art for VH domains and for VHH domains, the total number of amino acid residues in each of the CDRs may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, in general, the numbering according to Kabat may or may not correspond to the actual numbering of the amino acid residues in the actual sequence. Other numbering systems or numbering schemes include Chothia, IMGT, and AbM.

"Humanized antibody", also known as CDR-grafted antibody, refers to an antibody produced by grafting non-human CDR sequences into the framework of variable regions of a human antibody. Chimeric antibodies, due to their significant non-human protein content, can induce a strong immune response. This issue can be overcome by using humanized antibodies. To avoid the decrease in activity caused by the decrease in immunogenicity, the variable regions of a fully human antibody can be subjected to minimum reverse mutation to maintain activity. Examples of "humanization" include "humanization" of VHH domains derived from camelidae by replacing one or more amino acid residues in the amino acid sequence of the original VHH sequence with one or more amino acid residues present at the corresponding positions in a VH domain of a human conventional antibody with a four-peptide-chain structure (also referred to in the present disclosure as "sequence optimization"; in addition to humanization, "sequence optimization" may also encompass other modifications to the sequence by one or more mutations providing improved properties of the VHH, such as removal of potential post-translational modification sites). The humanized VHH domain may contain one or more fully human framework region sequences, and in some specific embodiments, may contain the human framework region sequence of IGHV3. Methods for humanization include, e.g., protein surface amino acid humanization (resurfacing) and universal framework grafting method for antibody humanization (CDR grafting to a universal framework), i.e., "grafting" CDRs onto other "frameworks" (including but not limited to human scaffolds or non-immunoglobulin scaffolds). Scaffolds and techniques suitable for such CDR grafting are known in the art. For example, germline DNA sequences of genes of the human heavy and light chain variable regions can be found in the VBase human species sequence database, as well as in Kabat, E. A. et al., 1991 Sequences of Proteins of Immunological Interest, 5th edition. The humanized antibody of the present disclosure also includes humanized antibodies which are further subjected to CDR avidity maturation by phage display. Additionally, in order to avoid the decrease in activity caused by the decrease in immunogenicity, the framework sequence in the human antibody variable region can be subjected to minimum reverse mutation or back mutation to maintain activity.

The term "affinity-matured" antibody is an antibody that has one or more changes in one or more hypervariable regions (HVRs) that result in an improvement in the affinity of the antibody for the antigen, as compared to the parent antibody which does not have such changes. For example, an "avidity-matured" IL-36R-binding protein or anti-IL-36R antibody has one or more changes in one or more CDRs that result in an increase in the avidity for the antigen as compared to its parent antibody. Avidity-matured antibodies can be prepared, for example, by methods known in the art as described below: Marks et al., 1992, Biotechnology 10:779-783 or Barbas et al., 1994, Proc. Nat. Acad. Sci, USA 91:3809-3813; Shier et al., 1995, Gene 169:147-155; Yelton et al., 1995, Immunol. 155:1994-2004; Jackson et al., 1995, J. Immunol. 154(7):3310-9; Hawkins et al., 1992, J. Mol. Biol. 226(3):889896; KS Johnson and RE Hawkins, "Affinity maturation of antibodies using phage display", Oxford University Press 1996.

Typically, the IL-36R-binding protein or the IL-36R/IL-23-binding protein of the present disclosure will bind to the antigen or target protein to be bound (i.e., IL-36R, IL-23, or CD16A) with a dissociation constant (K_{D}) of preferably 10⁻⁷ to 10⁻¹⁰ mol/L (M), more preferably 10⁻⁸ to 10⁻¹⁰ mol/L, and even more preferably 10⁻⁹ to 10⁻¹⁰ or less, and/or with an association constant (KA) of at least 10⁻⁷ M, preferably at least 10⁻⁸ M, more preferably at least 10⁻⁹ M, and more preferably at least 10⁻¹⁰ M, as measured in a Biacore or KinExA or Fortibio assay. Any K_{D} value greater than 10⁻⁴ M is generally considered to indicate non-specific binding. Specific binding of an antigen-binding protein to an antigen or epitope can be measured in any suitable manner known, including, for example, surface plasmon resonance (SPR) assay, Scatchard assay, and/or competitive binding assay (e.g., radioimmunoassay (RIA), enzyme immunoassay (EIA), and sandwich competitive assay) described in the present disclosure.

"Binding affinity" or "affinity" is used in the present disclosure as a measure of the strength of a non-covalent interaction between two molecules (e.g., an antibody or a portion thereof and an antigen). The binding affinity between two molecules can be quantified by determining the dissociation constant (K_{D}). K_{D} can be determined by measuring the kinetics of complex formation and dissociation by using, e.g., the surface plasmon resonance (SPR) method (Biacore). The rate constants corresponding to the association and dissociation of a monovalent complex are referred to as the association rate constant ka (or kon) and the dissociation rate constant kd (or koff), respectively. K_{D} is related to ka and kd by the equation K_{D} = kd/ka. The value of the dissociation constant can be determined directly by well-known methods, and calculations can even be performed for complex mixtures by, for example, those methods described in Caceci et al (1984, Byte 9:340-362). For example, K_{D} can be determined by using a dual filtration nitrocellulose filter binding assay such as that disclosed by Wong & Lohman (1993, Proc. Natl. Acad. Sci. USA 90:5428-5432). Other standard assays for evaluating the binding ability of an antibody to a target antigen are known in the art and include, for example, ELISA, western blot, RIA, and flow cytometry, as well as other assays exemplified elsewhere in the present disclosure. The binding kinetics and binding affinity of the antibody can also be evaluated by standard assays known in the art, such as surface plasmon resonance (SPR), e.g., by using the Biacore^{™} system or KinExA. The binding affinities associated with different molecular interactions, e.g., the binding affinities of different antibodies for a given antigen, can be compared by comparing the K_{D} values of antibody/antigen complexes. Similarly, the specificity of an interaction can be evaluated by determining and comparing the K_{D} value for the interaction of interest (e.g., a specific interaction between an antibody and an antigen) with the K_{D} value for an interaction not of interest (e.g., a control antibody known not to bind to IL-36R, IL-23, or CD16A).

"Conservative replacement" refers to a replacement with another amino acid residue having similar properties to the original amino acid residue. For example, lysine, arginine, and histidine have similar properties in that they have basic side chains, and aspartic acid and glutamic acid have similar properties in that they have acidic side chains. Additionally, glycine, asparagine, glutamine, serine, threonine, tyrosine, cysteine, and tryptophan have similar properties in that they have uncharged polar side chains, and alanine, valine, leucine, threonine, isoleucine, proline, phenylalanine, and methionine have similar properties in that they have nonpolar side chains. In addition, tyrosine, phenylalanine, tryptophan, and histidine have similar properties in that they have aromatic side chains. Thus, it will be apparent to those skilled in the art that even when an amino acid residue in a group exhibiting similar properties as described above is replaced, it will not exhibit a particular change in properties.

"Homology", "identity", or "sequence identity" refers to sequence similarity between two polynucleotide sequences or between two polypeptides. When positions in two compared sequences are occupied by identical nucleotides or amino acid monomers, e.g., if the position of each of two DNA molecules is occupied by an identical nucleotide, the molecules are homologous at that position. The homology percentage between two sequences is a function of the number of matching or homologous positions shared by the two sequences divided by the number of positions compared × 100%. For example, if 6 out of 10 positions are matched or homologous when two sequences are optimally aligned, the two sequences are 60% homologous. In general, when two sequences are aligned, a comparison is performed to obtain the maximum homology percentage.

"Nucleic acid" or "polynucleotide" are used interchangeably in the present disclosure to refer to any DNA or RNA molecule, either single- or double-stranded, and, if single-stranded, the molecule of its complementary sequence, preferably a double-stranded DNA. A nucleic acid is "operably linked" when it is placed into a functional relationship with another nucleic acid sequence. For example, a promoter or enhancer is operably linked to a coding sequence if it affects the transcription of the coding sequence.

"Host cell" includes individual cells or cell cultures which may be or have been the recipient of a vector for incorporation of a polynucleotide insert. The host cell includes progeny of a single host cell, and the progeny may not necessarily be completely identical (in morphology or genomic DNA complement) to the original parent cell due to natural, accidental, or deliberate mutations. The host cell includes cells transfected and/or transformed *in vivo* with the polynucleotide of the present disclosure. "Cell", "cell line", and "cell culture" are used interchangeably, and all such designations include their progeny. It should also be understood that all progeny may not be precisely identical in DNA content due to intentional or unintentional mutations. Mutant progeny with identical function or biological activity as those screened in the initially transformed cells are included.

"Inhibition" and "blocking" are used interchangeably and encompass both partial and complete inhibition/blocking. "Inhibition of growth" (e.g., involving cells) is intended to include any measurable reduction in cell growth.

"Giving", "administering", and "treating", when applied to animals, humans, experimental subjects, cells, tissues, organs, or biological fluids, refer to contact of an exogenous drug, a therapeutic agent, a diagnostic agent, or a composition with the animals, humans, subjects, cells, tissues, organs, or biological fluids, e.g., therapeutic, pharmacokinetic, diagnostic, research, and experimental methods. The treatment of cells comprises contacting a reagent with cells and contacting the reagent with a fluid, where the fluid is in contact with the cells. "Giving", "administering", and "treating" also refer to treating, e.g., a cell, by a reagent, diagnosis, a binding composition, or by another cell *in vitro* and *ex vivo.* When applied to humans, veterinary medicine, or research subjects, they refer to therapeutic treatment, preventive or prophylactic measures, and research and diagnostic applications.

"Treating" or "treatment" refers to administering a therapeutic agent, such as a therapeutic agent comprising any one of the binding proteins of the present disclosure or a pharmaceutical composition thereof, either internally or externally, to a subject who has had, is suspected of having, or is predisposed to having one or more proliferative diseases or symptoms thereof on which the therapeutic agent is known to have a therapeutic effect. Typically, the therapeutic agent is administered in an amount effective to alleviate one or more disease symptoms in the subject or population being treated, whether by inducing regression of such symptoms or inhibiting the development of such symptoms into any clinically measurable degree. The amount of therapeutic agent effective to alleviate any specific disease symptom (also referred to as the "therapeutically effective amount") may vary depending on a variety of factors such as the disease state, the age and weight of the subject, and the capability of the drug to produce a desired therapeutic effect in the subject. Whether a disease symptom has been alleviated can be evaluated by any clinical testing methods commonly used by doctors or other health care professionals to evaluate the severity or progression of the symptom. Although embodiments of the present disclosure (e.g., treatment methods or articles of manufacture) may be ineffective in alleviating a disease symptom of interest in a certain subject, they shall alleviate the disease symptom of interest in a statistically significant number of subjects as determined by any statistical test method known in the art, such as the Student's t-test, Chi-square test, U-test by Mann and Whitney, Kruskal-Wallis test (H-test), Jonckheere-Terpstra test, and Wilcoxon test.

"Effective amount" comprises an amount sufficient to ameliorate or prevent a symptom or sign of a medical disorder. An effective amount also means an amount sufficient to allow or facilitate diagnosis. The effective amount for a subject may vary depending on factors such as the disorder to be treated, the general health of the subject, the method, route, and dose of administration, and the severity of side effects. An effective amount may be the maximum dose or administration regimen to avoid significant side effects or toxic effects. The subject of the present disclosure may be an animal or a human subject. "Optional" or "optionally" means that the event or circumstance subsequently described may, but does not necessarily, occur, and this description includes instances where the event or circumstance occurs or does not occur. "And/or" should be taken as specific disclosure of each of the two specified features or components with or without the other. Thus, the term "and/or" as used in a phrase such as "A and/or B" in the present disclosure includes "A and B", "A or B", "A" (alone), and "B" (alone). Unless otherwise clearly stated in the context, throughout the description and the claims, the words "comprise", "have", "include", and the like are to be understood in an inclusive sense as opposed to an exclusive or exhaustive sense; that is to say, in the sense of "including, but not limited to". In the context of the mutations contained in the Fc region of the present disclosure, "/" represents "and"; for example, "L234A/L235A" represents "L234A and L235A"; that is, the Fc comprises mutations L234A and L235A; the amino acid positions of the mutations in the Fc region of the present disclosure are defined according to the EU numbering scheme.

The "subject" or "patient" in the present disclosure means a mammal, particularly a primate, and especially a human.

### BRIEF DESCRIPTION OF THE DRAWINGS

FIGs. 1A and 1B show the binding activity of anti-IL-36R single-domain antibodies VHH162 and VHH213 and humanized antibodies thereof to human IL-36R on the surface of CHO-K1 cells.
FIG. 2 shows the results of competitive binding between VHH162_Hu09, VHH213_Hu01, and control antibodies BI655130 and H4H14706P2.
FIG. 3A shows the experimental results of scores for the severity of erythema in imiquimod-induced psoriasis animals; FIG. 3B shows the experimental results of scores for the severity of skin scaling in imiquimod-induced psoriasis animals; FIG. 3C shows the results of the comprehensive scoring for erythema and skin scaling in imiquimod-induced psoriasis animals.
FIG. 4A shows the experimental results of the right ear thickness of human IL-23-induced psoriasis animals; FIG. 4B shows the experimental results of the area under the curve of human IL-23-induced psoriasis animals (in the figure, *** P < 0.001 compared to the negative control); FIG. 4C shows the experimental results of the right ear weight of human IL-23-induced psoriasis animals (in the figure, ** P < 0.01 and *** P < 0.001 compared to the negative control).
FIG. 5 shows a structural schematic diagram of the anti-IL-36R and anti-IL-23 bispecific antibodies of the present disclosure.
FIG. 6A shows the results of the binding activity of bispecific antibodies IL-36R-IL-23-01 and IL-36R-IL-23-02 to human IL-23 measured by ELISA; FIG. 6B shows the results of the binding activity of bispecific antibodies IL-36R-IL-23-01 and IL-36R-IL-23-02, as well as anti-IL-36R single-domain antibodies VHH162_Hu11 and VHH213_Hu07 to human IL-36R on the cell surface.
FIGs. 7A-7D show the inhibitory effects of IL-36R-IL-23-01 and IL-36R-IL-23-02 on IL-36-induced IL-8 secretion by A431.
FIGs. 8A and 8B show the inhibitory effects of IL-36R-IL-23-01 and IL-36R-IL-23-02 on the proliferation of BaF3-mIL-23R cells induced by IL-23.
FIG. 9 shows the inhibitory effects of IL-36R-IL-23-01 and IL-36R-IL-23-02 on the IL-23-induced DB-STAT3-Luc2 reporter gene system.
FIG. 10A shows a flowchart of the study on the human IL-36R transgenic mouse ear swelling model induced by human IL-36 and human IL-23; FIG. 10B shows the effects of IL-36R-IL-23-01 and VHH213_Hu07 on the ear weight in the mouse ear swelling model induced by human IL-36 and human IL-23; FIG. 10C shows the effects of IL-36R-IL-23-01 and VHH213_Hu07 on the downstream gene expression in the mouse ear swelling model induced by human IL-36 and human IL-23.
FIG. 11 shows the effects of IL-36R-IL-23-01 and VHH213_Hu07 on the downstream gene expression in the mouse ear swelling model induced by human IL-36 and human IL-23.

### DETAILED DESCRIPTION

The present disclosure is further described below with reference to examples, but these examples are not intended to limit the scope of the present disclosure. Experimental procedures without specific conditions specified in the examples of the present disclosure were generally conducted under conventional conditions such as those outlined in Antibodies: A Laboratory Manual and Molecular Cloning: A Laboratory Manual by Cold Spring Harbor Laboratory, or under conditions recommended by the manufacturers of the starting materials or commercial products. Reagents without specific sources indicated were commercially available conventional reagents.

### Example 1. Screening for Anti-IL-36R Antibodies

### 1. Immunizing antigen, screening antigen, and cell line construction

A human IL-36R extracellular domain (Novoprotein, CJ62) with a human Fc tag at the C-terminus was used as an immunizing antigen, and a human IL-36R extracellular domain (Kactus, IL1-HM4L2) with a his tag and an avi tag at the C-terminus, a biotinylated human IL-36R extracellular domain (Kactus, IL1-HM4L2B), or a cynomolgus monkey IL-36R extracellular domain (Kactus, IL1-CM1L2) with a his tag at the C-terminus was used as a screening antigen. The specific information on the immunizing antigen and screening antigens is shown in Table 1.

**Table 1. Information on IL-36R antigens for immunizing and screening**

| Antigen | Manufacturer and catalog No. | Starting and ending amino acids | Accession No. |
|---|---|---|---|
| Human IL-36R protein (Fc Tag) | Novoprotein, CJ62 | Asp20-Tyr337 | #Q9HB29-1 |
| Human IL-36R protein (His + Avi Tag) | Kactus, IL1-HM4L2 | Asp20-Arg335 | |
| Human IL-36R protein (His + Avi Tag, biotinylation) | Kactus, IL1-HM4L2B | | |
| Monkey IL-36R protein (His Tag) | Kactus, IL1-CM1L2 | Asp20-Arg335 | #EHH61702.1 |

A CHO-K1 cell line stably expressing human IL-36R and monkey IL-36R full-length proteins was constructed using the FlpIn system. FlpIn^{™}-CHO-K1 cells (Thermo, R75807) were plated at 2E5 cells/well in a six-well plate (culture medium: F12 + 10% FBS + 100 µg/mL Zeocin). After overnight culture, Lipo3000 (GIBCO, L3000001) was used to transfect a mixture of plasmids overexpressing human IL-36R or monkey IL-36R full-length protein and pOG44 (Thermo, V6005). After transfection for 24 h, the culture medium was replaced with a fresh one (culture medium: F12 + 10% FBS). After transfection for 48 h, the cells were digested, and 800 µg/mL hygromycin B (Gibco, 10687010) was added for resistance screening. After the negative cells completely disappeared, the remaining cells were digested, and a portion was taken for flow cytometry identification. Cells with a positive rate higher than 95% were expanded and cryopreserved for subsequent antibody binding screening.

The sequences of human and monkey IL-36R are shown below, with the sequences of the extracellular regions underlined-they start with the amino acid Asp20 and end with the amino acid Arg335.
> Human IL-36R sequence (Q9HB29-1) (SEQ ID NO: 1) 
MWSLLLCGLSIALPLSVTADGCKDIFMKNEILSASQPFAFNCTFPPITSGEVSVTW
YKNSSKIPVSKIIQSRIHQDETWILFLPMEWGDSGVYQCVIKGRDSCHRIHVNLT
VFEKHWCDTSIGGLPNLSDEYKQILHLGKDDSLTCHLHFPKSCVLGPIKWYKDC
NEIKGERFTVLETRLLVSNVSAEDRGNYACQAILTHSGKQYEVLNGITVSITERA
GYGGSVPKIIYPKNHSIEVQLGTTLIVDCNVTDTKDNTNLRCWRVNNTLVDDYY
DESKRIREGVETHVSFREHNLYTVNITFLEVKMEDYGLPFMCHAGVSTAYIILQL
PAPDFRAYLIGGLIALVAVAVSVVYIYNIFKIDIVLWYRSAFHSTETIVDGKLYDAY
VLYPKPHKESQRHAVDALVLNILPEVLERQCGYKLFIFGRDEFPGQAVANVIDEN
 
VKLCRRLIVIVVPESLGFGLLKNLSEEQIAVYSALIQDGMKVILIELEKIEDYTVM
PESIQYIKQKHGAIRWHGDFTEQSQCMKTKFWKTVRYHMPPRRCRPFPPVQLL
QHTPCYRTAGPELGSRRKKCTLTTG
> Monkey IL-36R (EHH61702.1) (SEQ ID NO: 2)

MWSLLLCGLSIALPLSVTADGCNNIFMKNEMLSASQPFAFNCTFPPITSGEVSVT
WYKNSSKIPVSKIIQSRIHQDETWILFLPMEWGDSGVYQCVIKGRDSCHRIHVNL
TVFEKQWCDTSVKGLPNLSDEYKQILHLGKDDSLTCHLHFPKSCILGPIKWYKD
CNEITGERFTVLETRLLVSNVSAEDRGNYACQVILTHSGRQYEVLNGITVSITER
AGYGGSVPKIIYPKNNSIEVQLGTTLIVDCNITDTKDNTNLRCWRVNNTLVDDY
YNESKRIREGVETHASFREHNLYTVNITFLEVKMEDYGLPFMCHAGVSAAYIML
QLPAPDFRAYLIGGLIAFLAVAVSVVYIYNIFKIDIVLWYRSTFQSTETLEDGKLYD
AYVLYPKPRRESQRHAVDTLVLKILPEVLERQCGYKLFIFGRDEFPGQAVANVID
ENVKLCRRLIVIVVPESLGFGLLKNLSEEQIAVYNALIQDGMKVILIELEKIEDYT
AMPESIQYIRQKHGVIRWHGDFTEQSQCVKTKFWKTVRYHMPPRRCRPSPPVQ
LPQHTPCYRTTASPKLGSRRKKCTLKTG

### 2. Camelid immunization, nanobody phage display library construction, and anti-human IL-36R nanobody screening

### 1) Camelid immunization

Two healthy camelids *(Camelus bactrianus)* were immunized with a human IL-36R extracellular domain (Novoprotein, CJ62) with a human Fc tag as an immunizing antigen. On day 0, the first immunization was performed: 200 µg of the human IL-36R antigen was well mixed with Freund's complete adjuvant (CFA), and the mixture was subcutaneously injected. On days 14, 28, and 42, 200 µg of the human IL-36R antigen was well mixed with Freund's incomplete adjuvant (IFA), and the mixture was subcutaneously injected. Blood samples were collected on days 42 and 56 for a serum titer assay. Nanobody phage display libraries were constructed separately for peripheral lymphocytes from the two camelids.

### 2) Phage library construction

After the fourth immunization of the 2 camelids, 50 mL of peripheral blood samples were taken. PBMCs were separated from the peripheral blood samples, and RNA was extracted from the PBMCs and reverse-transcribed to obtain total cDNA. Following amplification of the antibody genes using the camelid antibody feature sequence primers, VHH gene fragments were amplified using the VHH feature sequences and cloned into the phage library vector (pSCD-2). The ligation products were introduced into *E. coli* TG1 competent cells by electroporation. The library capacity was calculated to be 1.1 × 10⁹ through gradient dilution plating. After the transfected cells were infected with M13KO7 and cultured overnight, phage particles expressing VHH were released. The supernatant was collected, sterilized by filtration at 4 °C, and stored at -80 °C until further use.

### 3) Nanobody (VHH) screening

The VHH phage library was screened by liquid phase affinity panning, and biotinylated human IL-36R (Kactus, IL1-HM4L2B) was used as a screening antigen. The phage library was mixed with the antigen, and complexes of the human IL-36R antigen and the phage were captured using streptavidin-coupled agarose microspheres. After washing with PBS/0.05% Tween, the phages bound to the microspheres were eluted with a Glycine Elution Buffer (0.2 M Glycine-HCl, pH 2.2, 1 mg/mL BSA). After the phages obtained by elution were amplified, a second round of screening was performed. 10 µg/mL and 5 µg/mL human IL-36R antigens were used in the two rounds of screening, respectively. After two rounds of screening, clones were selected from the titer assay plate, and subjected to ELISA identification, sequencing, and sequence analysis to obtain the sequences VHH162 and VHH213 of the nanobodies binding to the human IL-36R antigen, which are shown below with the CDRs underlined, see Table 2.
> VHH162 (SEQ ID NO: 3) 
DVQLVESGGGSVQPGGSLRLSCAASGYSYRTQCLGWYRQAPGKQREAVATISG
GGTSTYYSDSVKGRFTISQDNARTTVYLSLTSLKPEDTATYYCAAGLSVLGSWC
HGGSSYEYTYSGQGTQVTVSS
> VHH213 (SEQ ID NO: 4)

EVQLVESGGGSVQAGGSLRLSCAASGNTYSRYCMGWFRQAPGKEREGVAAIDS
DGDTSYADSVEGRFTISKDNAKNTLYLQMNSLKPEDTAMYYCAADFTPSAPPR
MGSGGYCFGDYWGQGTQVTVSS

**Table 2. CDR sequences of anti-IL-36R nanobodies (defined according to the Kabat numbering scheme)**

| Name of nanobody | Heavy chain CDR | | Sequence No. |
|---|---|---|---|
| VHH162 | CDR1 | TQCLG | SEQ ID NO:5 |
| | CDR2 | TISGGGTSTYYSDSVKG | SEQ ID NO:6 |
| | CDR3 | GLSVLGSWCHGGSSYEYTY | SEQ ID NO:7 |
| VHH213 | CDR1 | RYCMG | SEQ ID NO:8 |
| | CDR2 | AIDSDGDTSYADSVEG | SEQ ID NO:9 |
| | CDR3 | DFTPSAPPRMGSGGYCFGDY | SEQ ID NO:10 |

The variable region sequences of the nanobodies in Table 2 were each linked to a human IgG4 Fc (comprising a hinge region and an S228P mutation, according to the EU numbering scheme) fragment to construct VHH-Fc antibodies. Plasmids were constructed and transiently transfected into HEK293 cells, and the antibodies were secreted and expressed. The antibodies in the supernatant were purified using a protein A column, and the column was washed with PBS and eluted with a 0.1 M glycine buffer (pH 2.5). The eluted protein was dialyzed against a PBS buffer at pH 7.4. The human IgG4 Fc sequence is as follows:
> hIgG4-Fc (comprising an S228P mutation) (SEQ ID NO: 11)

ESKYGPPCPPCPAPEFLGGPSVFLFPPKPKDTLMISRTPEVTCVVVDVSQEDPEV
QFNWYVDGVEVHNAKTKPREEQFNSTYRVVSVLTVLHQDWLNGKEYKCKVS
NKGLPSSIEKTISKAKGQPREPQVYTLPPSQEEMTKNQVSLTCLVKGFYPSDIAVE
WESNGQPENNYKTTPPVLDSDGSFFLYSRLTVDKSRWQEGNVFSCSVMHEALH
NHYTQKSLSLSLGK

### Example 2. Humanization of Anti-IL-36R Antibodies

Antibody humanization was performed by CDR-grafting. Through the website of IMGT or NCBI, the variable regions of the parent IL-36R antibody were aligned with fully human germline genes in the IMGT or NCBI/igblast database. A human germline gene highly homologous to the anti-IL-36R antibody was selected as a humanization template. The CDRs of the camelid-derived antibody were recombined with the framework regions of the corresponding humanization template to form a humanized sequence. Furthermore, residues in the FRs that affected the stability and affinity of the antibody were back-mutated, and a mutation design was performed on immunogenicity risk sites, deamidation and isomerization risk sites, and the like.

### 1. Humanized framework selection and back mutation design for VHH162

The humanized heavy chain templates of the camelid-derived nanobody VHH162 were IGHV3-23*04 and IGHJ1*01. The heavy chain CDRs of the camelid-derived nanobody were recombined with the framework regions of the corresponding humanization template to form a heavy chain variable region sequence of an antibody comprising humanized FR1-camelid-derived CDR1-humanized FR2-camelid-derived CDR2-humanized FR3-camelid-derived CDR3-humanized FR4, where FR1, FR2, and FR3 were derived from IGHV3-23*04, and FR4 was derived from IGHJ1*01. The resulting humanized variable region sequence (the underlined parts represent CDR sequences, and the italicized parts represent FR sequences, defined according to the Kabat numbering scheme) is as follows:
> VHH162-graft (humanized VHH) (SEQ ID NO: 12)

EVQLVESGGGLVQPGGSLRLSCAASGFTFSTQCLGWVRQAPGKGLEWVSTISGGGT
STYYSDSVKGRFTISRDNSKNTLYLQMNSLRAEDTAVYYCAKGLSVLGSWCHGGSS
YEYTYWGQGTLVTVSS

On the basis of VHH 162-graft, a back mutation design was performed. The back mutations are shown in Table 3-1.

**Table 3-1. Back mutation design for VHH162 (sites defined according to the Kabat numbering scheme)**

| Antibody No. | Back mutation |
|---|---|
| VHH162-Hu00 | E1D, F27Y, T28S, F29Y, S30R, V37Y, G44Q, L45R, W47A, S49A, R71Q, S74A, N76T, L78V, K94A, W103S |
| VHH162-Hu01 | E1D, F27Y, T28S, F29Y, S30R, V37Y, G44Q, W47A, S49A, R71Q, S74A, N76T, L78V, K94A, W103S |
| VHH162-Hu02 | E1D, F27Y, T28S, F29Y, S30R, V37Y, G44Q, L45R, W47A, S49A, R71Q, S74A, N76T, L78V, K94A |
| VHH162-Hu03 | E1D, F27Y, T28S, F29Y, S30R, V37Y, G44Q, L45R, W47A, S49A, S74A, N76T, L78V, K94A, W103S |
| VHH162-Hu04 | E1D, F27Y, T28S, F29Y, S30R, V37Y, G44Q, L45R, W47A, S49A, R71Q, S74A, L78V, K94A, W103S |
| VHH162-Hu05 | E1D, F27Y, T28S, F29Y, S30R, V37Y, G44Q, L45R, W47A, S49A, R71Q, S74A, N76T, K94A, W103S |
| VHH162-Hu06 | E1D, F27Y, T28S, F29Y, S30R, V37Y, G44Q, L45R, W47A, S49A, R71Q, S74A, N76T, L78V, W103S |
| VHH162-Hu07 | F27Y, T28S, F29Y, S30R, V37Y, G44Q, L45R, W47A, S49A, R71Q, S74A, N76T, L78V, K94A, W103S |
| VHH162-Hu08 | E1D, F27Y, T28S, F29Y, S30R, V37Y, G44Q, L45R, W47A, R71Q, S74A, N76T, L78V, K94A, W103S |
| VHH162-Hu09 | E1D, F27Y, T28S, F29Y, S30R, V37Y, G44Q, L45R, W47A, S49A, R71Q, N76T, L78V, K94A, W103S |
| VHH162-Hu10 | F27Y, T28S, F29Y, S30R, V37Y, G44Q, W47A |
| VHH162-Hu11 | F27Y, T28S, F29Y, S30R, V37Y, G44Q, L45R, W47A, S49A, R71Q, N76T, L78V, W103S |
| VHH162-Hu12 | F27Y, T28S, F29Y, S30R, V37Y, G44Q, L45R, W47A, S49A, L78V, W103S |
| VHH162-Hul3 | F27Y, T28S, F29Y, S30R, V37Y, G44Q, L45R, W47A, L78V |

The humanized sequences of the VHH162 after the back mutation (the underlined parts represent CDR sequences, defined according to the Kabat numbering scheme) are as follows:
> VHH162_Hu00 (SEQ ID NO: 13) 
DVQLVESGGGLVQPGGSLRLSCAASGYSYRTQCLGWYRQAPGKQREAVATISG
GGTSTYYSDSVKGRFTISQDNAKTTVYLQMNSLRAEDTAVYYCAAGLSVLGSW
CHGGSSYEYTYSGQGTLVTVSS
> VHH162_Hu01 (SEQ ID NO: 14) 
DVQLVESGGGLVQPGGSLRLSCAASGYSYRTQCLGWYRQAPGKQLEAVATISG
GGTSTYYSDSVKGRFTISQDNAKTTVYLQMNSLRAEDTAVYYCAAGLSVLGSW
CHGGSSYEYTYSGQGTLVTVSS
> VHH162 Hu02 (SEQ ID NO: 15) 
DVQLVESGGGLVQPGGSLRLSCAASGYSYRTQCLGWYRQAPGKQREAVATISG
GGTSTYYSDSVKGRFTISQDNAKTTVYLQMNSLRAEDTAVYYCAAGLSVLGSW
CHGGSSYEYTYWGQGTLVTVSS
> VHH162_Hu03 (SEQ ID NO: 16) 
DVQLVESGGGLVQPGGSLRLSCAASGYSYRTQCLGWYRQAPGKQREAVATISG
GGTSTYYSDSVKGRFTISRDNAKTTVYLQMNSLRAEDTAVYYCAAGLSVLGSW
CHGGSSYEYTYSGQGTLVTVSS
> VHH162_Hu04 (SEQ ID NO: 17) 
DVQLVESGGGLVQPGGSLRLSCAASGYSYRTQCLGWYRQAPGKQREAVATISG
GGTSTYYSDSVKGRFTISQDNAKNTVYLQMNSLRAEDTAVYYCAAGLSVLGS
WCHGGSSYEYTYSGQGTLVTVSS
> VHH162_Hu05 (SEQ ID NO: 18) 
DVQLVESGGGLVQPGGSLRLSCAASGYSYRTQCLGWYRQAPGKQREAVATISG
GGTSTYYSDSVKGRFTISQDNAKTTLYLQMNSLRAEDTAVYYCAAGLSVLGSW
CHGGSSYEYTYSGQGTLVTVSS
> VHH162_Hu06 (SEQ ID NO: 19) 
DVQLVESGGGLVQPGGSLRLSCAASGYSYRTQCLGWYRQAPGKQREAVATISG
GGTSTYYSDSVKGRFTISQDNAKTTVYLQMNSLRAEDTAVYYCAKGLSVLGSW
CHGGSSYEYTYSGQGTLVTVSS
> VHH162_Hu07 (SEQ ID NO: 20) 
EVQLVESGGGLVQPGGSLRLSCAASGYSYRTQCLGWYRQAPGKQREAVATISG
GGTSTYYSDSVKGRFTISQDNAKTTVYLQMNSLRAEDTAVYYCAAGLSVLGSW
CHGGSSYEYTYSGQGTLVTVSS
> VHH162_Hu08 (SEQ ID NO: 21) 
DVQLVESGGGLVQPGGSLRLSCAASGYSYRTQCLGWYRQAPGKQREAVSTISG
GGTSTYYSDSVKGRFTISQDNAKTTVYLQMNSLRAEDTAVYYCAAGLSVLGSW
CHGGSSYEYTYSGQGTLVTVSS
> VHH162_Hu09 (SEQ ID NO: 22) 
DVQLVESGGGLVQPGGSLRLSCAASGYSYRTQCLGWYRQAPGKQREAVATISG
GGTSTYYSDSVKGRFTISQDNSKTTVYLQMNSLRAEDTAVYYCAAGLSVLGSW
CHGGSSYEYTYSGQGTLVTVSS
> VHH162_Hu10 (SEQ ID NO: 23) 
EVQLVESGGGLVQPGGSLRLSCAASGYSYRTQCLGWYRQAPGKQLEAVSTISG
GGTSTYYSDSVKGRFTISRDNSKNTLYLQMNSLRAEDTAVYYCAKGLSVLGSW
CHGGSSYEYTYWGQGTLVTVSS
> VHH162_Hu11 (SEQ ID NO: 24) 
EVQLVESGGGLVQPGGSLRLSCAASGYSYRTQCLGWYRQAPGKQREAVATISG
GGTSTYYSDSVKGRFTISQDNSKTTVYLQMNSLRAEDTAVYYCAKGLSVLGSW
CHGGSSYEYTYSGQGTLVTVSS
> VHH162_Hu12 (SEQ ID NO: 25) 
EVQLVESGGGLVQPGGSLRLSCAASGYSYRTQCLGWYRQAPGKQREAVATISG
GGTSTYYSDSVKGRFTISRDNSKNTVYLQMNSLRAEDTAVYYCAKGLSVLGSW
CHGGSSYEYTYSGQGTLVTVSS
> VHH162_Hu13 (SEQ ID NO: 26)

EVQLVESGGGLVQPGGSLRLSCAASGYSYRTQCLGWYRQAPGKQREAVSTISG
GGTSTYYSDSVKGRFTISRDNSKNTVYLQMNSLRAEDTAVYYCAKGLSVLGSW
CHGGSSYEYTYWGQGTLVTVSS

The CDR sequences of the humanized and back-mutated VHH162 described above are identical to the CDR sequences of the camelid-derived VHH162.

### 2. Humanized framework selection and back mutation design for VHH213

The humanized heavy chain templates of the camelid-derived nanobody VHH213 were IGHV3-74*01 and IGHJ1*01, FR1, FR2, and FR3 were derived from IGHV3-74*01, and FR4 was derived from IGHJ1*01. The following sequences were obtained:
> VHH213-graft (humanized VHH) (SEQ ID NO: 27)

EVQLVESGGGLVQPGGSLRLSCAASGFTFSRYCMGWVRQAPGKGLVWVSAIDSDG
DTSYADSVEGRFTISRDNAKNTLYLQMNSLRAEDTAVYYCARDFTPSAPPRMGSGG
YCFGDYWGQGTLVTVSS

On the basis of VHH213-graft, a back mutation design was performed. The back mutations are shown in Table 3-2.

**Table 3-2. Back mutation design for VHH213 (sites defined according to the Kabat numbering scheme)**

| Antibody No. | Back mutation |
|---|---|
| VHH213-Hu00 | F27N, F29Y, V37F, G44E, L45R, W47G, S49A, R71K, R94A |
| VHH213-Hu01 | F27N, F29Y, V37F, G44E, W47G, S49A, R71K, R94A |
| VHH213-Hu02 | F27N, F29Y, V37F, G44E, L45R, W47G, S49A, R94A |
| VHH213-Hu03 | F27N, F29Y, V37F, G44E, L45R, W47G, S49A, R71K |
| VHH213-Hu04 | F27N, F29Y, V37F, G44E, L45R, W47G, R71K, R94A |
| VHH213-Hu05 | F27N, F29Y, V37F, G44E, W47G |
| VHH213-Hu06 | F27N, F29Y, V37F, G44E, W47G, S49A, R94A |
| VHH213-Hu07 | F27N, V37F, G44E, W47G, S49A, R94A |

The humanized sequences of the VHH213 after the back mutation (the underlined parts represent CDR sequences, defined according to the Kabat numbering scheme) are as follows:
> VHH213_Hu00 (SEQ ID NO: 28) 
EVQLVESGGGLVQPGGSLRLSCAASGNTYSRYCMGWFRQAPGKERVGVAAIDS
DGDTSYADSVEGRFTISKDNAKNTLYLQMNSLRAEDTAVYYCAADFTPSAPPR
MGSGGYCFGDYWGQGTLVTVSS
> VHH213_Hu01 (SEQ ID NO: 29) 
EVQLVESGGGLVQPGGSLRLSCAASGNTYSRYCMGWFRQAPGKELVGVAAIDS
DGDTSYADSVEGRFTISKDNAKNTLYLQMNSLRAEDTAVYYCAADFTPSAPPR
MGSGGYCFGDYWGQGTLVTVSS
> VHH213_Hu02 (SEQ ID NO: 30) 
EVQLVESGGGLVQPGGSLRLSCAASGNTYSRYCMGWFRQAPGKERVGVAAIDS
DGDTSYADSVEGRFTISRDNAKNTLYLQMNSLRAEDTAVYYCAADFTPSAPPR
MGSGGYCFGDYWGQGTLVTVSS
> VHH213_Hu03 (SEQ ID NO: 31) 
EVQLVESGGGLVQPGGSLRLSCAASGNTYSRYCMGWFRQAPGKERVGVAAIDS
DGDTSYADSVEGRFTISKDNAKNTLYLQMNSLRAEDTAVYYCARDFTPSAPPR
MGSGGYCFGDYWGQGTLVTVSS
> VHH213_Hu04 (SEQ ID NO: 32) 
EVQLVESGGGLVQPGGSLRLSCAASGNTYSRYCMGWFRQAPGKERVGVSAIDS
DGDTSYADSVEGRFTISKDNAKNTLYLQMNSLRAEDTAVYYCAADFTPSAPPR
MGSGGYCFGDYWGQGTLVTVSS
> VHH213_Hu05 (SEQ ID NO: 33) 
EVQLVESGGGLVQPGGSLRLSCAASGNTYSRYCMGWFRQAPGKELVGVSAIDS
DGDTSYADSVEGRFTISRDNAKNTLYLQMNSLRAEDTAVYYCARDFTPSAPPRM
GSGGYCFGDYWGQGTLVTVSS
> VHH213_Hu06 (SEQ ID NO: 34) 
EVQLVESGGGLVQPGGSLRLSCAASGNTYSRYCMGWFRQAPGKELVGVAAIDS
DGDTSYADSVEGRFTISRDNAKNTLYLQMNSLRAEDTAVYYCAADFTPSAPPR
MGSGGYCFGDYWGQGTLVTVSS
> VHH213_Hu07 (SEQ ID NO: 35)

EVQLVESGGGLVQPGGSLRLSCAASGNTFSRYCMGWFRQAPGKELVGVAAIDS
DGDTSYADSVEGRFTISRDNAKNTLYLQMNSLRAEDTAVYYCAADFTPSAPPR
MGSGGYCFGDYWGQGTLVTVSS

The CDR sequences of the humanized and back-mutated VHH213 sequences are identical to the CDR sequences of the camelid-derived VHH213.

The humanized and back-mutated sequences of VHH162 and VHH213 were linked to a human IgG4 Fc (comprising a hinge region and an S228P mutation, according to the EU numbering scheme) fragment to construct VHH-Fc antibodies. The humanized antibody recombinant plasmids were constructed and then transiently transfected into HEK293 cells, and the antibodies were secreted and expressed. The antibodies in the supernatant were purified using a protein A column, and the column was washed with PBS and eluted with a 0.1 M glycine buffer (pH 2.5). The eluted protein was dialyzed against a PBS buffer at pH 7.4. Analysis showed that the antibodies of interest were obtained.

### Example 3. Identification of Affinity of Humanized Anti-IL-36R Antibodies for IL-36R Antigen

### 1. Assay on binding of humanized anti-IL-36R antibodies to IL-36R on cell surface by FACS

To assay the binding ability of the humanized anti-IL-36R antibodies to human IL-36R expressed on the cell membrane, the candidate anti-IL-36R antibodies at different concentrations were added to CHO-K1 cells (1E5 cells/well) overexpressing human IL-36R, and the cells were incubated on ice for 1 h. After the cells were washed twice with a FACS buffer (1% FBS, DPBS), 100 µL of fluorescent secondary antibody AF488-goat anti-human IgG (H + L) (1:1000, Invitrogen, A11013) was added, and the cells were incubated on ice for 30 min. The cells were washed twice with a FACS buffer and resuspended in 150 µL of a FACS buffer. Fluorescence signals were detected on a flow cytometer (BD, FACSCelesta), and the EC₅₀ was calculated.

The anti-human IL-36R antibody BI655130 (WO2013074569A1, obtained by self-expression according to the sequence in the patent) from Boehringer Ingelheim and the anti-human IL-36R antibody H4H14706P2 (WO2020018503A2, obtained by self-expression according to the sequence in the patent) from Regeneron were used as controls. The sequences are as follows:
> B1655130 heavy chain sequence (SEQ ID NO: 36) 
QVQLVQSGAEVKKPGASVKVSCKASGYSFTSSWIHWVKQAPGQGLEWMGEIN
PGNVRTNYNENFRNKVTMTVDTSISTAYMELSRLRSDDTAVYYCTVVFYGEPYF
PYWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
RVEPKSCDKTHTCPPCPAPEAAGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSREEMTKNQVSLTCLVKGFYP
SDIAVEWESNGQPENNYKTTPPVLDSDGSFFLYSKLTVDKSRWQQGNVFSCSVM
HEALHNHYTQKSLSLSPGK
> BI655130 light chain sequence (SEQ ID NO: 37) 
QIVLTQSPGTLSLSPGERATMTCTASSSVSSSYFHWYQQKPGQAPRLWIYRTSRL
ASGVPDRFSGSGSGTDFTLTISRLEPEDAATYYCHQFHRSPLTFGAGTKLEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
> H4H14706P2 heavy chain sequence (SEQ ID NO: 38) 
EVQLVESGGGLVQPGRSLRLSCTASGFTFDDYAIHWVRQSPGKGLEWVSVISWN
SDVIGYADSVKGRFTISRDNAKNSLYLQMNSLRAEDTALYYCAKGYNWNFFDY
WGQGTLVTVSSASTKGPSVFPLAPCSRSTSESTAALGCLVKDYFPEPVTVSWNS
GALTSGVHTFPAVLQSSGLYSLSSVVTVPSSSLGTKTYTCNVDHKPSNTKVDKR
VESKYGPPCPPCPAPEFLGGPSVFLFPPKPKDTLMISRTPEVTCVVVDVSQEDPE
 
VQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIAV
EWESNGQPENNYKTTPPVLDSDGSFFLYSRLTVDKSRWQEGNVFSCSVMHEAL
HNHYTQKSLSLSLGK
> H4H14706P2 light chain sequence (SEQ ID NO: 39)

EIVLTQSPATLSLSPGERATLSCRASQSVSSYLAWYQQKPGQAPRLLIYNAANRA
TDIPARFSGSGSGTDFTLTISSLEPEDFAVYYCQQRSNWPLTFGGGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

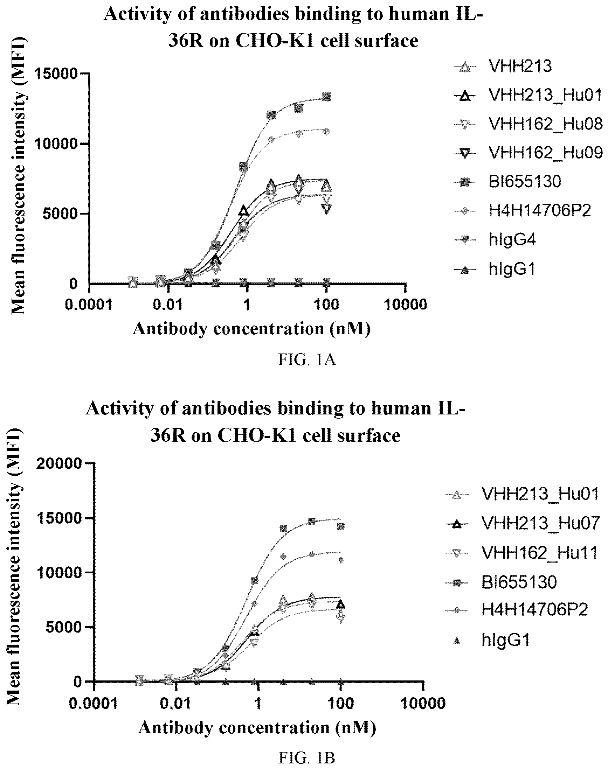

The results of the binding of the VHH162 humanized antibodies VHH162_Hu08, VHH162_Hu09, and VHH162_Hu11, and the VHH213 humanized antibodies VHH213_Hu01 and VHH213_Hu07 to human IL-36R are shown in FIGs. 1A and 1B and Tables 4-1 and 4-2. VHH162_Hu08, VHH162_Hu09, VHH162_Hu11, VHH213_Hu01, and VHH213_Hu07 have relatively strong binding activity to human IL-36R on the surface of CHO-Kl cells, and their binding activity is comparable to that of VHH213 to human IL-36R on the surface of CHO-K1 cells.

**Table 4-1. EC₅₀ values for the binding of VHH162 and VHH213 humanized antibodies to human IL-36R measured by FACS**

| Antibody No. | Binding activity | |
|---|---|---|
| | EC₅₀ (nM) | Maximum MFI |
| VHH213 | 0.57 | 6920 |
| VHH213_Hu01 | 0.40 | 7069 |
| VHH162_Hu08 | 0.66 | 6009 |
| VHH162_Hu09 | 0.48 | 5336 |
| BI655130 | 0.51 | 13355 |
| H4H14706P2 | 0.37 | 10877 |
| hIgG1 | N.A. | 77 |
| hIgG4 | N.A. | 76 |

| | | |
|---|---|---|
| Note: N.A. represents not applicable | | |

**Table 4-2. EC₅₀ values for the binding of VHH162 and VHH213 humanized antibodies to human IL-36R measured by FACS**

| Antibody No. | Binding activity | |
|---|---|---|
| | EC₅₀ (nM) | Maximum MFI |
| VHH213_Hu01 | 0.41 | 7674 |
| VHH213_Hu07 | 0.53 | 7780 |
| VHH162_Hu11 | 0.63 | 6947 |
| BI655130 | 0.51 | 14233 |
| H4H14706P2 | 0.51 | 11152 |
| hIgG1 | N.A. | 47 |

| | | |
|---|---|---|
| Note: N.A. represents not applicable | | |

### 2. Assay on binding of humanized anti-IL-36R antibodies to IL-36R antigen by SPR

The affinities of the IL-36R antibodies for the IL-36R antigen were measured using surface plasmon resonance (SPR).

Biacore^{™} 8k (GE Healthcare) was used. In the experiment, a CM5 sensor chip (Cat. # 29149603, GE) was used, and an HBS-EP + buffer (10 mM HEPES, 150 mM NaCl, 3 mM EDTA, 0.05% surfactant P20) was used as a mobile phase. A 30 µg/mL solution of anti-human IgG (Fc) antibody was prepared in a 10 mM sodium acetate buffer (pH 5.0), and the Immobilization program was selected to automatically perform anti-human IgG (Fc) antibody channel amino coupling immobilization. The test antibodies were separately diluted in an HBS-EP + buffer as ligands and captured using the anti-human IgG (Fc) antibody on the chip channels. The human IL-36R antigen protein (Kactus, IL1-HM4L2) was used as an analyte. The analyte was serially diluted 2-fold with an HBS-EP + buffer, and the dilution was made to flow through the experimental and reference channels at a flow rate of 30 µL/min, with association for 1 min and dissociation for 15 min. The regeneration buffer 10 mM Glycine pH 1.5 (GE Healthcare, BR-1003-54) was run at a flow rate of 10 µL/min for 30 s. The association rate Ka, the dissociation rate Kd, and the dissociation constant (i.e., avidity K_{D}) were calculated. The results are shown in Table 5.

**Table 5. SPR affinity data for anti-IL-36R antibodies binding to human IL-36R antigen**

| Antibody No. | Antigen | ka (1/Ms) | kd (1/s) | K_{D} (M) |
|---|---|---|---|---|
| VHH162_Hu11 | Human IL-36R antigen | 4.70E+03 | 1.46E-04 | 3.11E-08 |
| VHH213_Hu07 | Human IL-36R antigen | 1.14E+04 | 9.39E-05 | 8.26E-09 |
| BI655130 | Human IL-36R antigen | 1.33E+04 | 9.54E-05 | 7.16E-09 |
| H4H14706P2 | Human IL-36R antigen | 5.68E+04 | 3.64E-04 | 6.40E-09 |

The results of the binding to the human IL-36R antigen show that VHH162_Hu11 and VHH213_Hu07 have relatively strong affinities for human IL-36R, and their affinities are similar to those of BI655130 and H4H14706P2 for human IL-36R.

### 3. Results of the competitive binding of humanized anti-IL-36R antibodies with control antibodies

The competitive binding analysis of the anti-IL-36R antibodies to the human IL-36R protein was determined using a Biacore^{™} 8k (GE Healthcare) instrument. In the experiment, a CM5 sensor chip was used, and an HBS-EP + buffer (10 mM HEPES, 150 mM NaCl, 3 mM EDTA, 0.05% surfactant P20) was used as a mobile phase. According to the method in the instructions of a His capture kit (Cat. # 29234602, Cytiva) and an amine coupling kit (Cat. # BR100633, GE), an anti-His antibody was covalently coupled to a CM5 biosensor chip (Cat. # 29149603, GE) to affinity-capture the human IL-36R protein (Kactus, IL1-HM4L2). Subsequently, antibody 1 was injected until saturation, i.e., the signal no longer increased. Antibody 2 was then injected. The reaction signals were detected in real time using the Biacore^{™} 8k instrument to obtain a binding curve. After the dissociation in each cycle was completed, the biochip was washed and regenerated using 10 mM Glycine pH 1.5 (GE Healthcare, BR-1003-54). The data obtained from the experiment were fitted with the Tandem using dual model by using the Biacore Insight Evaluation 3.0 software.

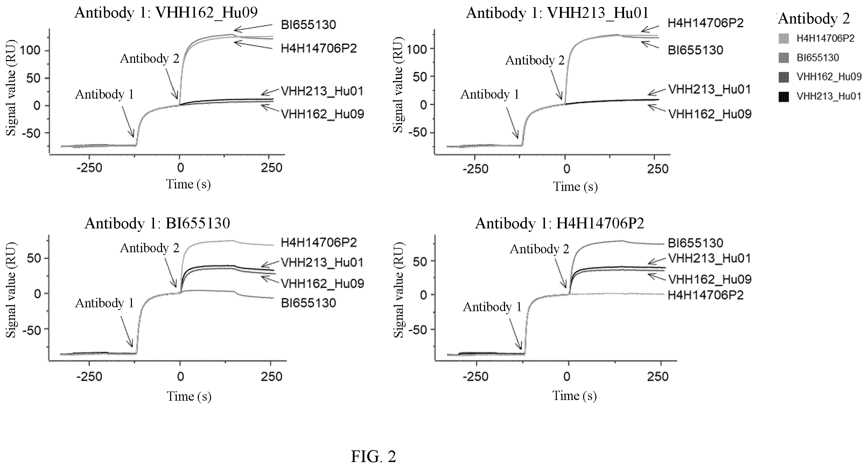

The results are shown in FIG. 2. The antigenic epitopes of VHH162_Hu09 and VHH213_Hu01 overlap and differ from those of H4H14706P2 and B1655130.

### Example 4. Preparation of Anti-Human IL-23 p19 Monoclonal Antibodies

### 1. Preparation of murine antibody

SJL mice were immunized with a human IL-23 protein (Sino Biological, CT048-H08H). After 3 rounds of immunization, blood was collected to measure the serum antibody titers. Mice with high and plateauing serum antibody titers were selected for spleen cell fusion. The fused hybridoma cells were plated in a 96-well cell culture plate and cultured in a 5% CO₂ incubator at 37 °C. The cell culture supernatant was taken for detection by ELISA. The positive clones obtained by screening were expanded, cryopreserved, and subcloned until single cell clones were obtained. The selected hybridoma clones were further subjected to serum-free cell culture to prepare and purify antibodies. The obtained hybridoma antibodies were subjected to ELISA assay for detecting the binding of the antibodies to the human IL-23 protein and the blocking of the receptors by the antibodies (see Example 5 of the present disclosure), and hybridoma cell strains with good binding activity and blocking activity were selected.

The monoclonal hybridoma cell strain mAb29 was selected. The cloning process was as follows: hybridoma cells in the logarithmic growth phase were collected, and the RNA was extracted using Trizol (Invitrogen, Cat. # 15596-018) and reverse-transcribed into cDNA. PCR amplification was performed using the cDNA as a template, and the products were sent to a sequencing company for sequencing. The amino acid sequences of the variable regions of the antibodies corresponding to the obtained DNA sequences are as follows. The CDR sequences are shown in Table 6.
> mAb29 heavy chain variable region (SEQ ID NO: 40) 
QVQLKESGPGLVAPSQSLSITCTVSGFSLTSYAISWVRQPPGKGLEWLGVIWTGG
GTNYNSGIKSRLTISKDNSKSQVFLNMNSLQTEDTARYYCVREGDYGSYAGAM
DYWGQGTSVTVSS
> mAb29 light chain variable region (SEQ ID NO: 41)

DIVLTQSPASLAVSLGQRATISCRASESVDYYGISSMHWFQQKPGQPPKLLIYRAS
NLESGIPARFSGSGSRTDFTLTINRVETDDVATYYCQQANKDPYTFGGGTKLEIK

**Table 6. Heavy and light chain CDR sequences of mAb29 (defined according to the kabat numbering scheme)**

| Heavy chain variable region CDR | | Light chain variable region CDR | |
|---|---|---|---|
| HCDR1 | SYAIS (SEQ ID NO:42) | LCDR1 | RASESVDYYGISSMH (SEQ ID NO:45) |
| HCDR2 | VIWTGGGTNYNSGIKS (SEQ ID NO:43) | LCDR2 | RASNLES (SEQ ID NO:46) |
| HCDR3 | EGDYGSYAGAMDY (SEQ ID NO:44) | LCDR3 | QQANKDPYT (SEQ ID NO:47) |

The variable region sequences of mAb29 described above were PCR amplified for VH/VK sequences, respectively, which were subjected to homologous recombination with the expression vector pHr (with signal peptide and hIgG1/hkappa constant region gene (CH1-Fc/CL) fragments). Exemplarily, the human heavy chain IgG1 constant region sequence is set forth in SEQ ID NO: 48, the human light chain κ constant region sequence is set forth in SEQ ID NO: 49, a recombinant chimeric antibody full-length expression plasmid VH-CH1-Fc-pHr/VL-CL-pHr was constructed, and thereby chimeric antibody Ch29 was obtained.
> Human IgG1 heavy chain constant region (comprising L234A/L235A mutations) (SEQ ID NO: 48) 
ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPEAAGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSL
SLSPGK
> Human light chain κ constant region (SEQ ID NO: 49)

### 2. Humanization

By comparing with the IMGT human antibody heavy and light chain variable region germline gene database, heavy and light chain variable region germline genes with high homology were selected as templates, and CDRs of the murine antibody were grafted into corresponding humanized templates to form variable region sequences in the following order: FR1-CDR1-FR2-CDR2-FR3-CDR3-FR4, and then the variable region sequences were fused to human constant region sequences to obtain the humanized antibody. Humanization of mAb29 is described below exemplarily, in which the CDR amino acid residues of the antibodies are determined and annotated by the Kabat numbering scheme. Heavy and light chain variable region germline genes with high homology were selected as templates. IGKV4-1 *01 and IGKJ4*01 were selected as the template of the humanized light chain of the murine antibody mAb29, that is, FR1, FR2, and FR3 of the human germline light chain IGKV4-1*01 and a JK4 region (as FR4) of IGKJ4*01 were selected as the humanized antibody light chain framework regions; IGHV2-26*01 and IGHJ6*01 were selected as the template of the humanized heavy chain, that is, FR1, FR2, and FR3 of the human germline heavy chain IGHV2-26*01 and a JH6 region (as FR4) of IGHJ6*01 were selected as the humanized antibody light chain framework regions. Firstly, CDRs of the murine antibody mAb29 were grafted into selected corresponding humanization templates to replace the CDR regions of the humanization templates; and then, in the humanized antibody, the amino acid residues at positions 4, 17, 36, 58, 60, and/or 68 (numbering according to the Kabat numbering scheme) of the light chain variable region and the amino acid residues at positions 1, 30, 37, 44, 49, 73, 89, and/or 93 (numbering according to the Kabat numbering scheme) of the heavy chain variable region were mutated. In addition, the amino acid residue at position 1 of the HCDR1: SYAIS (SEQ ID NO: 42) of the heavy chain variable region was mutated to N or Q, resulting in a novel HCDR1: NYAIS (SEQ ID NO: 50) or QYAIS (SEQ ID NO: 51). The variable region sequences of the humanized antibody of mAb29 are as follows:
> hAb29VH1 (Graft + Q1E, A49G, and A93V) (SEQ ID NO: 52) 
EVTLKESGPVLVKPTETLTLTCTVSGFSLSSYAISWIRQPPGKALEWLGVIWTGG
GTNYNSGIKSRLTISKDTSKSQVVLTMTNMDPVDTATYYCVREGDYGSYAGAM
 DYWGQGTTVTVSS
> hAb29VH2 (Graft + Q1E, S30T, A49G, T73N, and A93V) (SEQ ID NO: 53) 
EVTLKESGPVLVKPTETLTLTCTVSGFSLTSYAISWIRQPPGKALEWLGVIWTGG
GTNYNSGIKSRLTISKDNSKSQVVLTMTNMDPVDTATYYCVREGDYGSYAGAM
DYWGQGTTVTVSS
> hAb29VH3 (Graft + Q1E, S30T, I37V, A49G, T73N, T89R, and A93V) (SEQ ID NO: 54) 
EVTLKESGPVLVKPTETLTLTCTVSGFSLTSYAISWVRQPPGKALEWLGVIWTGG
GTNYNSGIKSRLTISKDNSKSQVVLTMTNMDPVDTARYYCVREGDYGSYAGAM
DYWGQGTTVTVSS
> hAb29VH4 (Graft + S30T, I37V, A44G, A49G, T73N, T89R, and A93V) (SEQ ID NO: 55) 
QVTLKESGPVLVKPTETLTLTCTVSGFSLTSYAISWVRQPPGKGLEWLGVIWTGG
GTNYNSGIKSRLTISKDNSKSQVVLTMTNMDPVDTARYYCVREGDYGSYAGAM
DYWGQGTTVTVSS
> hAb29VH5 (Graft + S30T, I37V, A44G, A49G, T73N, T89R, and A93V + S31N) (SEQ ID NO: 56) 
QVTLKESGPVLVKPTETLTLTCTVSGFSLTNYAISWVRQPPGKGLEWLGVIWTG
GGTNYNSGIKSRLTISKDNSKSQVVLTMTNMDPVDTARYYCVREGDYGSYAGA
MDYWGQGTTVTVSS
> hAb29VH6 (Graft + S30T, I37V, A44G, A49G, T73N, T89R, and A93V + S31Q) (SEQ ID NO: 57) 
QVTLKESGPVLVKPTETLTLTCTVSGFSLTQYAISWVRQPPGKGLEWLGVIWTG
GGTNYNSGIKSRLTISKDNSKSQVVLTMTNMDPVDTARYYCVREGDYGSYAGA
MDYWGQGTTVTVSS
> hAb29VL1 (Graft + Y36F and G68R) (SEQ ID NO: 58) 
DIVMTQSPDSLAVSLGERATINCRASESVDYYGISSMHWFQQKPGQPPKLLIYRA
SNLESGVPDRFSGSGSRTDFTLTISSLQAEDVAVYYCQQANKDPYTFGGGTKVEI
K
> hAb29VL2 (Graft + M4L, Y36F, V58I, and G68R) (SEQ ID NO: 59) 
DIVLTQSPDSLAVSLGERATINCRASESVDYYGISSMHWFQQKPGQPPKLLIYRA
SNLESGIPDRFSGSGSRTDFTLTISSLQAEDVAVYYCQQANKDPYTFGGGTKVEI
K
> hAb29VL3 (Graft + M4L, Y36F, V58I, D60A, and G68R) (SEQ ID NO: 60) 
DIVLTQSPDSLAVSLGERATINCRASESVDYYGISSMHWFQQKPGQPPKLLIYRA
SNLESGIPARFSGSGSRTDFTLTISSLQAEDVAVYYCQQANKDPYTFGGGTKVEIK
> hAb29VL4 (Graft + M4L, E17Q, Y36F, V58I, D60A, and G68R) (SEQ ID NO: 61)

DIVLTQSPDSLAVSLGQRATINCRASESVDYYGISSMHWFQQKPGQPPKLLIYRA
SNLESGIPARFSGSGSRTDFTLTISSLQAEDVAVYYCQQANKDPYTFGGGTKVEIK

Note: the sequence order is FR1-CDR1-FR2-CDR2-FR3-CDR3-FR4; in the sequences, the underlined parts represent CDR regions (CDR amino acid residues are determined by the Kabat numbering scheme), and the remainder represents FR regions.

Expression vectors of the light chain and the heavy chain of an antibody were constructed; cross-pairing combination was performed on the light/heavy chains of a humanized antibody, and the culture supernatant was collected and purified after 293E cells were transfected to obtain the humanized full-length antibody. The heavy chain constant region of the humanized antibody may be selected from the group consisting of IgG1, IgG2, IgG3, and IgG4 constant regions. Exemplarily, the aforementioned heavy chain variable region of the humanized antibody of mAb29 was fused to the human heavy chain IgG1 constant region (with the sequence set forth in SEQ ID NO: 48) to form an antibody full-length heavy chain, and the light chain variable region of the humanized antibody was fused to the human light chain κ constant region (with the sequence set forth in SEQ ID NO: 49) to form an antibody full-length light chain, resulting in the humanized antibodies of mAb29 shown in Table 7 below:

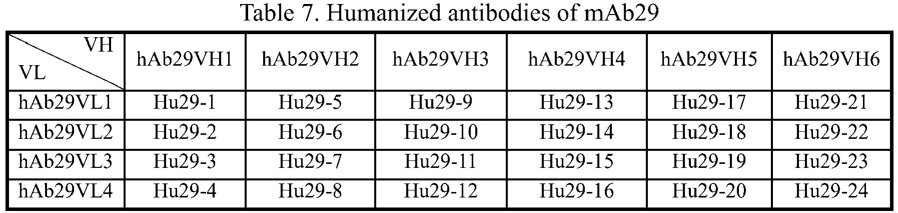

Exemplarily, heavy and light chain full-length sequences of humanized antibodies are as follows:
> Hu29-19 heavy chain (SEQ ID NO: 62) 
QVTLKESGPVLVKPTETLTLTCTVSGFSLTNYAISWVRQPPGKGLEWLGVIWTG
GGTNYNSGIKSRLTISKDNSKSQVVLTMTNMDPVDTARYYCVREGDYGSYAGA
MDYWGQGTTVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKKVEPK
SCDKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLSLSPG
K
> Hu29-19 light chain (SEQ ID NO: 63) 
DIVLTQSPDSLAVSLGERATINCRASESVDYYGISSMHWFQQKPGQPPKLLIYRA
SNLESGIPARFSGSGSRTDFTLTISSLQAEDVAVYYCQQANKDPYTFGGGTKVEIK
RTVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
> Hu29-24 heavy chain (SEQ ID NO: 64) 
QVTLKESGPVLVKPTETLTLTCTVSGFSLTQYAISWVRQPPGKGLEWLGVIWTG
GGTNYNSGIKSRLTISKDNSKSQVVLTMTNMDPVDTARYYCVREGDYGSYAGA
MDYWGQGTTVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKKVEPK
SCDKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLSLSPG
K
> Hu29-24 light chain (SEQ ID NO: 65)

DIVLTQSPDSLAVSLGQRATINCRASESVDYYGISSMHWFQQKPGQPPKLLIYRA
SNLESGIPARFSGSGSRTDFTLTISSLQAEDVAVYYCQQANKDPYTFGGGTKVEIK
RTVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

Note: the italicized parts represent constant regions, and the underlined parts represent mutations.

### Example 5. Functional Validation of Anti-Human IL-23 p19 Monoclonal Antibodies

### 1. Affinity assay

### 1) ELISA binding assay

The binding activity of the test molecules to IL-23 p19 and BAFF was measured by ELISA (coating the test molecules). The specific procedures were as follows:
The test sample was diluted to 2 µg/mL with a PBS buffer at pH 7.4 and added to a 96-well microplate at 100 µL/well. The plate was incubated at 4 °C overnight. After the liquid was discarded, 5% skim milk (BD, 232100), diluted with PBS, was added at 300 µL/well for blocking, and the plate was incubated at 37 °C for 2 h. After the blocking was completed, the blocking solution was discarded, and the plate was washed 3 times with a PBST buffer (pH 7.4, PBS containing 0.1% tween-20). The IL-23 (Sino Biological, CT048-H08H) or BAFF (ACROBiosystems, BAF-H52D4) solution diluted in a gradient was added at 100 µL/well, and the plate was incubated at 37 °C for 1 h. After the incubation was completed, the plate was washed 3 times with PBST. When the binding to IL-23 and BAFF was to be detected, Anti-His-HRP (Sino biological, 105327-MM02T-H, diluted at 1:2000) was added at 100 µL/well, and the plate was incubated at 37 °C for 1 h. After the plate was washed 3 times with PBST, the TMB chromogenic substrate (KPL, 5120-0077) was added at 100 µL/well. The plate was incubated at room temperature for 10-15 min, and 1 M H₂SO₄ was added at 50 µL/well to terminate the reaction. The absorbance values at 450 nm were measured using a microplate reader. Curves for the binding of the antibodies to the antigen were fitted using software, and EC₅₀ values were calculated. RCT-18 was telitacicept (RC18, RemeGen). The experimental results of the binding of the anti-IL-23 antibodies and the fusion protein thereof to human IL-23 are shown in Table 8-1.

**Table 8-1. Experimental results of binding to IL-23 protein**

| Sample | Binding activity EC₅₀ for human IL-23 (nM) | Binding activity EC₅₀ for human BAFF (nM) |
|---|---|---|
| Ch29 | 1.155 | - |
| Hu29-15 | 1.189 | - |
| Hu29-19 | 1.107 | - |
| Hu29-24 | 1.218 | - |
| RCT-18 | - | 21.49 |

The experimental results show that the anti-IL-23 antibodies of the present disclosure can specifically bind to the human IL-23 protein.

### 2) Biacore assay

A certain amount of test sample was subjected to affinity capture by a biosensor chip Protein A (GE, 29127556), and then antigens at a series of concentration gradients were allowed to flow on the surface of the chip. The reaction signals were detected in real time by using Biacore (GE, 8K) to obtain association and dissociation curves. After the dissociation in each cycle was completed, the biochip was washed and regenerated with a 10 mM glycine-hydrochloric acid solution at pH 1.5 (GE, BR-1003-54). The experimental data were fitted using BIAevaluation version 4.1 software with a 1:1 model to obtain affinity values. The related antigen protein used in this assay is as follows: human IL-23 (Sino biological, CT048-H08H). The test results of the affinity are shown in Table 8-2 and Table 8-3.

**Table 8-2. Experimental results of affinity for binding of anti-IL-23 antibodies to human IL-23 p19**

| Sample | Human IL-23 p19 | | |
|---|---|---|---|
| | ka (1/Ms) | kd (1/s) | K_{D} (M) |
| Hu29-19 | 2.71E+06 | 4.08E-05 | 1.51E-11 |
| Hu29-24 | 4.71E+06 | 7.03E-05 | 1.49E-11 |
| Risankizumab | 7.54E+05 | 4.91E-05 | 6.51E-11 |

**Table 8-3. Experimental results of affinity for binding of other anti-IL-23 antibodies to human IL-23 p19**

| Antibody | Human IL-23 p19 | | |
|---|---|---|---|
| | ka (1/Ms) | kd (1/s) | K_{D} (M) |
| Hu29-1 | 3.39E+06 | 1.33E-04 | 3.93E-11 |
| Hu29-2 | 3.51E+06 | 1.08E-04 | 3.08E-11 |
| Hu29-5 | 3.40E+06 | 1.25E-04 | 3.67E-11 |
| Hu29-6 | 3.86E+06 | 9.85E-05 | 2.55E-11 |
| Hu29-9 | 3.48E+06 | 1.06E-04 | 3.04E-11 |
| Hu29-10 | 3.65E+06 | 8.70E-05 | 2.39E-11 |
| Hu29-15 | 3.15E+06 | 7.02E-05 | 2.23E-11 |
| Hu29-16 | 4.36E+06 | 7.99E-05 | 1.83E-11 |
| Hu29-23 | 3.79E+06 | 8.57E-05 | 2.26E-11 |
| Ch29 | 4.05E+06 | 8.40E-05 | 2.07E-11 |

The experimental results show that the anti-IL-23 antibodies constructed in the present disclosure can bind to human IL-23 p19 with high affinity.

The sequences of the anti-IL-23 antibody control molecule risankizumab (see WHO Drug Information Vol. 30, No. 1, 2016) in the present disclosure are shown below:
> Risankizumab heavy chain (SEQ ID NO: 66) 
QVQLVQSGAEVKKPGSSVKVSCKASGYTFTDQTIHWMRQAPGQGLEWIGYIYP
RDDSPKYNENFKGKVTITADKSTSTAYMELSSLRSEDTAVYYCAIPDRSGYAWFI
YWGQGTLVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKR
VEPKSCDKTHTCPPCPAPEAAGGPSVFLFPPKPKDTLMISRTPEVTCVVVDVSHE
DPEVKFNWYVDGVEVHNAKTKPREEQYNSTYRVVSVLTVLHQDWLNGKEYK
CKVSNKALPAPIEKTISKAKGQPREPQVYTLPPSREEMTKNQVSLTCLVKGFYPS
 
DIAVEWESNGQPENNYKTTPPVLDSDGSFFLYSKLTVDKSRWQQGNVFSCSVM
HEALHNHYTQKSLSLSPG
> Risankizumab light chain (SEQ ID NO: 67)

DIQMTQSPSSLSASVGDRVTITCKASRDVAIAVAWYQQKPGKVPKLLIYWASTR
HTGVPSRFSGSGSRTDFTLTISSLQPEDVADYFCHQYSSYPFTFGSGTKLEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

### 2. Experiment on blocking of ligand and receptors

The blocking activity of the anti-IL-23 antibodies against IL-23/IL-23R was measured by ELISA. The specific procedures were as follows:
The receptor protein was diluted to 2 µg/mL with a PBS (BasalMedia, B320) buffer at pH 7.4 and added to a 96-well microplate (Corning, 3590) at 100 µL/well. The plate was incubated at 4 °C overnight. After the liquid was discarded, a 1% Casein blocking solution (Thermo, 37528) was added at 200 µL/well for blocking, and the plate was incubated at 37 °C for 2 h. After the blocking was completed, the blocking solution was discarded, and the plate was washed 3 times with a PBST buffer (pH 7.4, PBS containing 0.1% tween-20) and set aside for later use. A biotinylated ligand protein at a fixed concentration was mixed with a serially diluted antibody or fusion protein, and the mixture was preincubated at 37 °C for 30 min and then added to the blocked microplate. The plate was incubated at 37 °C for 1.5 h. After the incubation was completed, the plate was washed 3 times with PBST. Streptavidin-HRP (Invitrogen, 434323, diluted at 1:4000) was added at 100 µL/well, and the plate was incubated at 37 °C for 1 h. The supernatant was removed. After the plate was washed 3 times with PBST, the TMB chromogenic substrate (KPL, 5120-0077) was added at 100 µL/well. The plate was incubated at room temperature for 10-15 min, and 1 M H₂SO₄ was added at 50 µL/well to terminate the reaction. The absorbance values at 450 nm were measured using a microplate reader. Curves for the inhibition of binding of the ligand to the receptor were fitted using software, and the IC₅₀ values were calculated. The source information of the ligand protein and receptor proteins used in this test example is as follows: IL-23 (Sino Biological, CT048-H08H), IL-23R (Sino Biological, 13840-H02H), and BAFF-R (Sino biological, 16079-H02H).

The experimental results of the blocking of the anti-IL-23 antibodies against IL-23/IL-23R are shown in Tables 9-1 and 9-2.

**Table 9-1. Experimental results of blocking of anti-IL-23 antibodies and fusion protein thereof against IL-23/IL-23R**

| Sample | Blocking activity IC₅₀ for IL-23/IL-23R (nM) |
|---|---|
| Ch29 | 0.3858 |
| Hu29-19 | 0.4783 |
| Hu29-24 | 0.4877 |
| Risankizumab | 0.6104 |

**Table 9-2. Experimental results of blocking of other anti-IL-23 antibodies against IL-23/IL-23R**

| Sample | Blocking activity IC₅₀ for IL-23/IL-23R (nM) |
|---|---|
| Hu29-1 | 0.4743 |
| Hu29-2 | 0.3972 |
| Hu29-5 | 0.3881 |
| Hu29-6 | 0.4367 |
| Hu29-9 | 0.3966 |
| Hu29-10 | 0.4242 |
| Hu29-15 | 0.4104 |

The experimental results show that the anti-IL-23 antibodies constructed in the present disclosure can effectively block IL-23/IL-23R binding.

### 3. Experiment on proliferation of BaF3-IL-23R cells

The *in vitro* activity of the antibodies was detected by the experiment on proliferation of BaF3-IL-23R cells. The experimental procedures were as follows:
BaF3 cells (Cobioer, CBP60474) stably expressing IL-23R and IL-12Rβ1 were plated in a 96-well cell plate (Corning, 3903) at 50 µL/well, i.e., 4000 cells. The test sample diluted in a gradient was mixed with an IL-23 protein (Sino Biological, CT048-H08H) at a fixed concentration, and the mixture was added to the cell culture plate. The resulting mixture was cultured in an incubator at 37 °C for 72 h. The cell culture plate was then taken out, and the Celltiter Glo detection solution (Promega, G755B) was added at 50 µL/well. The plate was incubated in a shaker for 10 min and then taken out and left to stand at room temperature for 10 min, and bioluminescent signals were detected using a microplate reader (PerkinElmer, Victor3). Based on the data, inhibition curves were fitted using software, and IC₅₀ values were calculated.

The experimental results of the inhibition of proliferation of BaF3-IL-23R cells by anti-IL-23 antibodies are shown in Tables 10-1 and 10-2.

**Table 10-1. Experimental results of inhibition of proliferation of BaF3-IL-23R cells by anti-IL-23 antibodies and fusion protein thereof**

| Sample | Activity in inhibiting proliferation of BaF3-IL-23R cells (IC₅₀, nM) |
|---|---|
| Ch29 | 0.1753 |
| Hu29-19 | 0.1938 |
| Hu29-24 | 0.1784 |

**Table 10-2. Experimental results of inhibition of proliferation of BaF3-IL-23R cells by other anti-IL-23 antibodies**

| Sample | Activity in inhibiting proliferation of BaF3-IL-23R cells (IC₅₀, nM) |
|---|---|
| Hu29-1 | 0.07111 |
| Hu29-2 | 0.06227 |
| Hu29-5 | 0.05531 |
| Hu29-6 | 0.08897 |
| Hu29-9 | 0.05608 |
| Hu29-10 | 0.08601 |
| Hu29-15 | 0.2158 |
| Hu29-16 | 0.2251 |
| Hu29-23 | 0.1184 |

The experimental results show that the anti-IL-23 antibodies of the present disclosure all have strong activity in inhibiting the proliferation of BaF3-IL-23R cells.

### 4. IL-17 secretion assay

The IL-17 secretion assay was performed to detect the inhibition of the IL-23-induced T cell differentiation by the anti-IL-23 antibodies. The experimental procedures were as follows:
100 µL of 2 µg/mL anti-mouse CD3 antibody (BioLegend, 100238) and 2 µg/mL anti-mouse CD28 antibody (BioLegend, 102116) were added to each well of a 96-well plate (Corning, 3599). The plate was incubated at 37 °C for 1 h and then washed twice with PBS for later use. The spleens of mice were ground and centrifuged at 4 °C for 5 min, and lower-layer cells were collected, washed once with a washing solution (PBS + 2% FBS + 2 mM EDTA), and centrifuged. The supernatant was removed, the RBC Lysis Buffer (Invitrogen, 00-4333-57) was added, and the mixture was left to stand at room temperature for 5 min until the red blood cells were completely lysed. The lysate was centrifuged, and the cells were resuspended and counted. The cell suspension was sorted with the Mouse CD4 Cells Kit (Invitrogen, 11415D), and the isolated CD4+ T cells were resuspended in a culture medium of RPMI 1640 Medium (Gibco, 11875119) + 10% FBS (Gibco, 10099-141) and counted for later use. The cell suspension was plated on a coated 96-well plate. IL-23 (R&D Systems, 1290-IL-010) at a fixed concentration was mixed with the antibody or fusion protein diluted in a gradient, and the mixture was preincubated for 1 h and then added to the 96-well plate, and the resulting mixture was cultured in a cell incubator at 37 °C for 48 h. The 96-well plate was taken out and centrifuged at 1000 rpm for 3 min. The supernatant was collected and assayed for the IL-17 content using the Mouse IL-17 DuoSet ELISA Kit (R&D Systems, DY421). The experimental results are shown in Table 11.

**Table 11. Experimental results of inhibiting IL-17 secretion**

| Sample | IC₅₀ for inhibiting IL-17 secretion (nM) |
|---|---|
| Hu29-15 | 0.01954 |
| Hu29-19 | 0.01775 |
| Hu29-24 | 0.02890 |

The experimental results show that the anti-IL-23 antibodies of the present disclosure all have strong activity in inhibiting IL-17 secretion.

### 5. Imiquimod-induced psoriasis animal model experiment

The *in vivo* efficacy of anti-IL-23 antibodies was assessed by an imiquimod cream (IMQ)-induced psoriasis animal model experiment.

SPF-grade female C57BL/6 hIL-23A/hIL12B transgenic mice (Biocytogen Jiangsu Co., Ltd.) aged 8-9 weeks were randomly grouped with 5 mice in each group. The mice were depilated at the back prior to the start of the experiment. From day 0 to day 5, mice in the sham group were subjected to even application of 80 mg of vaseline (Unilever) on the skin of the back; mice in the animal model groups were subjected to even application of 80 mg of an imiquimod cream (Best Medicine Store) on the skin of the back for 6 consecutive days. The test samples (risankizumab, Hu29-19, Hu29-24, and Hu38-4 all at a dose of 20 mpk) were intraperitoneally injected 1 h prior to application of the imiquimod cream on day 0 and day 3. Risankizumab was used as a positive control and PBS was used as a negative control for a total of 2 administrations. The mice were scored daily for the severity of erythema and scaling of the back skin and the experiment ended on day 6. The scoring criteria for the severity of erythema were as follows: no erythema, 0 points; reddish, 1 point; red but not dark, 2 points; deep red, 3 points; extremely red, 4 points. The scoring criteria for scaling were as follows: no scaling, 0 points; a small area of tiny pieces of skin, 1 point; a moderate area of relatively thick pieces of skin, 2 points; a relatively larger area of coarse and thickened pieces of skin, 3 points; a large area of large pieces of skin, 4 points.

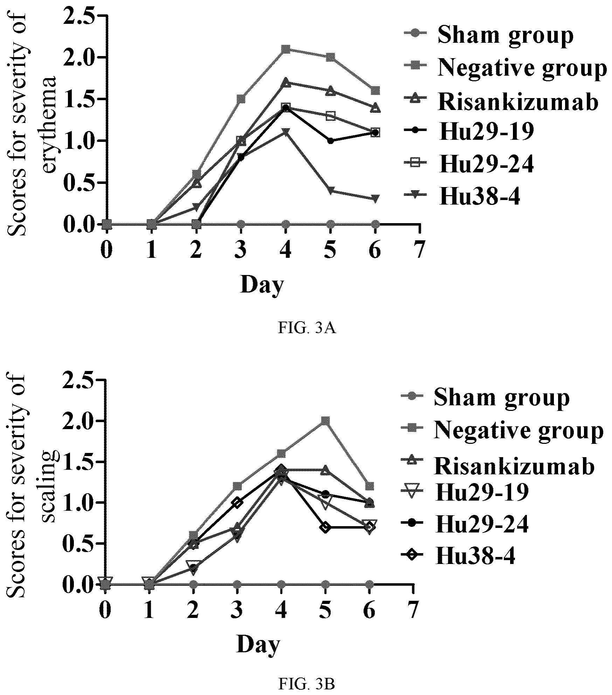

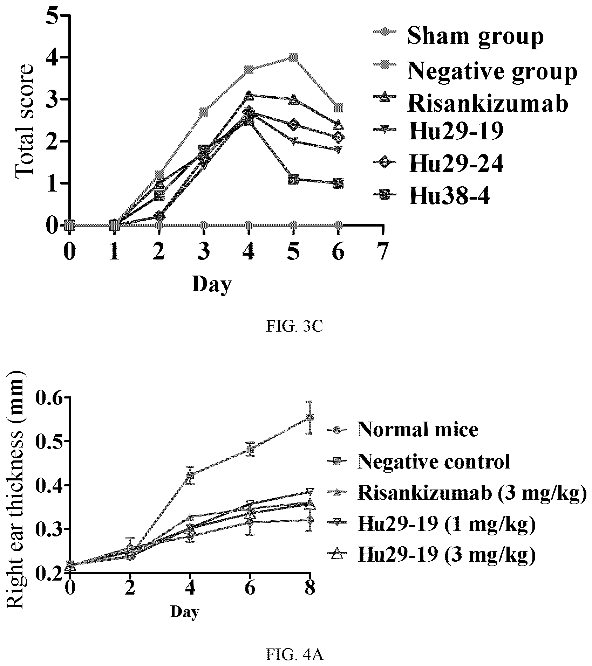

The experimental results are shown in FIGs. 3A-3C. The experimental results show that compared to the positive control risankizumab, the anti-IL-23 antibodies constructed in the present disclosure have stronger *in vivo* efficacy, and the scores of the severity of erythema and the severity of skin scaling are lower than those of risankizumab.

### 6. Human IL-23-induced psoriasis animal model experiment

The *in vivo* efficacy of anti-IL-23 antibodies constructed in the present disclosure was evaluated by a human IL-23-induced psoriasis animal model experiment. SPF-grade female C57BL/6J mice (Vital River Laboratory Animal Technology Co., Ltd.) aged 6-8 weeks were anesthetized, and then the right ear thickness and the body weight of the mice were measured. The mice were grouped according to the right ear thickness and body weight data, with 6 mice in each group. The day of grouping was recorded as day 0. From day 1, the mice were intradermally injected at the right ear with 1 µg of a human IL-23 protein (R&D Systems, 1290-IL-500/CF) daily, for 7 days in total, and the mice in the normal group were injected with PBS. The test drugs were intraperitoneally injected on day 0 and day 3, and the body weight and the right ear thickness of the mice were measured and recorded on days 0, 2, 4, 6, and 8. The alleviation effect of the test drugs on the model was evaluated by the change in the right ear thickness. The experiment ended on day 8, and the ears with a diameter of 8 mm were collected and weighed.

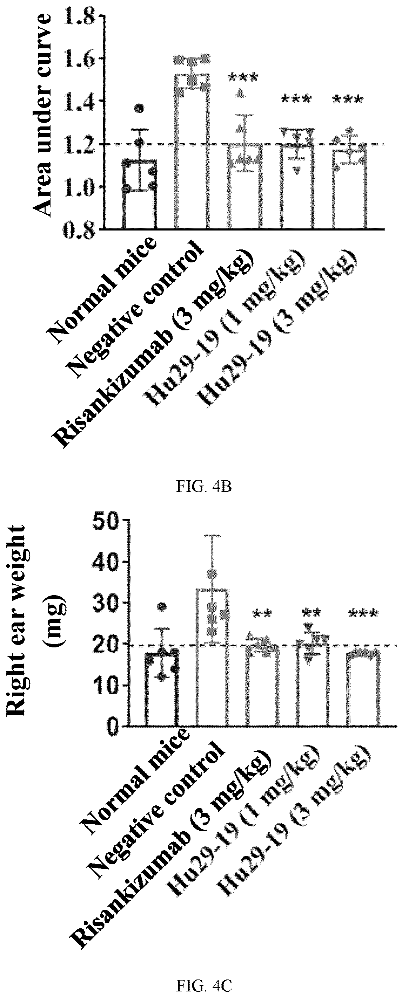

The experimental results are shown in FIGs. 4A-4C. The experimental results show that the *in vivo* efficacy of the anti-IL-23 antibody Hu29-19 constructed in the present disclosure in the human IL-23-induced psoriasis animal model is stronger than that of the control molecule risankizumab, where: when the doses were all 3 mpk, the ear thickness of the Hu29-19 group was lower than that of the control risankizumab; the statistical results of the area under the curve show that Hu29-19 exhibited a stronger effect than risankizumab at a dose of 3 mpk; statistical results of the mouse ear weight show that: the ear weight in the Hu29-19 1 mpk group was the same as that in the risankizumab 3 mpk group, and the ear weight in the Hu29-19 3 mpk group was lower than that in the risankizumab.

### Example 6. Construction of Anti-IL-36R and Anti-IL-23 Bispecific Antibodies

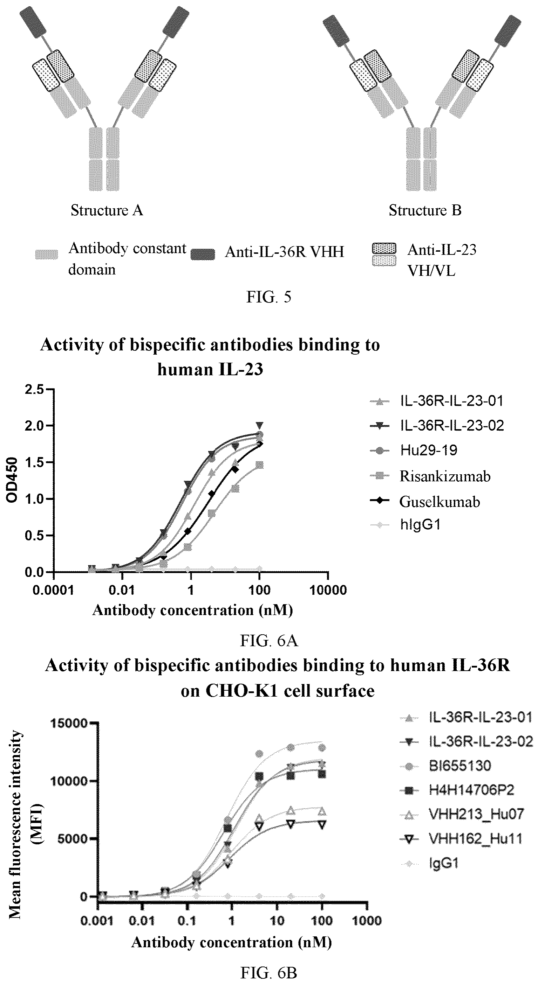

For the construction of bispecific molecules, the anti-IL-23 antibody Hu29-19 (with variable region sequences of hAb29VH5 (SEQ ID NO: 56) and hAb29VL3 (SEQ ID NO: 60)), and the humanized anti-human IL-36R nanobody sequence VHH213_Hu07 (SEQ ID NO: 35) or VHH162_Hu11 (SEQ ID NO: 24) were used to construct anti-IL-36R and anti-IL-23 bispecific antibody molecules with the human IgG1 heavy chain constant region and human kappa light chain constant region sequences according to the molecular format schematic diagram in FIG. 5. In the molecular formats represented by structure A and structure B in FIG. 5, the anti-IL-36R VHH sequences were fused to the N-termini of the heavy and light chains of the anti-IL-23 antibody, respectively, and linked via (G₄S)₂ linkers. The heavy chain constant regions of the constructed anti-IL-36R and anti-IL-23 bispecific antibodies comprised L234A and L235A mutations (according to the EU numbering system). The names and construction methods of the bispecific antibodies are shown in Table 12.

**Table 12. Construction rules and names of anti-IL-36R and anti-IL-23 bispecific antibodies**

| Bispecific antibody No. | Anti-IL-36R nanobody sequence | Anti-IL-23 antibody variable region sequence | Construction method |
|---|---|---|---|
| IL-36R-IL-23-01 | VHH213_Hu07 | Hu29-19 (hAb29VH5 and hAb29VL3) | Structure A |
| IL-36R-IL-23-02 | VHH162_Hu11 | | Structure A |
| IL-36R-IL-23-03 | VHH213_Hu07 | | Structure B |
| IL-36R-IL-23-04 | VHH162_Hu11 | | Structure B |

In the following sequences, the underlined parts represent linkers, and the italicized parts represent constant region sequences.
> IL-36R-IL-23-01 heavy chain (SEQ ID NO: 68) 
EVQLVESGGGLVQPGGSLRLSCAASGNTFSRYCMGWFRQAPGKELVGVAAIDS
DGDTSYADSVEGRFTISRDNAKNTLYLQMNSLRAEDTAVYYCAADFTPSAPPR
MGSGGYCFGDYWGQGTLVTVSSGGGGSGGGGSQVTLKESGPVLVKPTETLTLT
CTVSGFSLTNYAISWVRQPPGKGLEWLGVIWTGGGTNYNSGIKSRLTISKDNSKS
QVVLTMTNMDPVDTARYYCVREGDYGSYAGAMDYWGQGTTVTVSSASTKGPS
VFPLAPSSKSTSGGTAALGCLVKDYFPEPVTVSWNSGALTSGVHTFPAVLQSSGLYSLS
SVVTVPSSSLGTQTYICNVNHKPSNTKVDKKVEPKSCDKTHTCPPCPAPEAAGGPSVF
LFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSR
EEMTKNQVSLTCLVKGFYPSDIAVEWESNGQPENNYKTTPPVLDSDGSFFLYSKLTV
DKSRWQQGNVFSCSVMHEALHNHYTQKSLSLSPGK
> IL-36R-IL-23-01 light chain (SEQ ID NO: 69) 
DIVLTQSPDSLAVSLGERATINCRASESVDYYGISSMHWFQQKPGQPPKLLIYRA
SNLESGIPARFSGSGSRTDFTLTISSLQAEDVAVYYCQQANKDPYTFGGGTKVEIK
RTVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
> IL-36R-IL-23-02 heavy chain (SEQ ID NO: 70) 
EVQLVESGGGLVQPGGSLRLSCAASGYSYRTQCLGWYRQAPGKQREAVATISG
GGTSTYYSDSVKGRFTISQDNSKTTVYLQMNSLRAEDTAVYYCAKGLSVLGSW
CHGGSSYEYTYSGQGTLVTVSSGGGGSGGGGSQVTLKESGPVLVKPTETLTLTC
TVSGFSLTNYΛISWVRQPPGKGLEWLGVIWTGGGTNYNSGIKSRLTISKDNSKS
QVVLTMTNMDPVDTARYYCVREGDYGSYAGAMDYWGQGTTVTVSSASTKGPS
VFPLAPSSKSTSGGTAALGCLVKDYFPEPVTVSWNSGALTSGVHTFPAVLQSSGLYSLS
SVVTVPSSSLGTQTYICNVNHKPSNTKVDKKVEPKSCDKTHTCPPCPAPEAAGGPSVF
 
LFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSR
EEMTKNQVSLTCLVKGFYPSDIAVEWESNGQPENNYKTTPPVLDSDGSFFLYSKLTV
DKSRWQQGNVFSCSVMHEALHNHYTQKSLSLSPGK
> IL-36R-IL-23-02 light chain
   is set forth in SEQ ID NO: 69.
> IL-36R-IL-23-03 heavy chain (SEQ ID NO: 71) 
QVTLKESGPVLVKPTETLTLTCTVSGFSLTNYAISWVRQPPGKGLEWLGVIWTG
GGTNYNSGIKSRLTISKDNSKSQVVLTMTNMDPVDTARYYCVREGDYGSYAGA
MDYWGQGTTVTVSSASTKGPSVFPLAPSSKSTSGGTAALGCLVKDYFPEPVTVSWN
SGALTSGVHTFPAVLQSSGLYSLSSVVTVPSSSLGTQTYICNVNHKPSNTKVDKKVEPK
SCDKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAP
IEKTISKAKGQPREPQVYTLPPSREEMTKNQVSLTCLVKGFYPSDIAVEWESNGQPEN
NYKTTPPVLDSDGSFFLYSKLTVDKSRWQQGNVFSCSVMHEALHNHYTQKSLSLSPG
K
> IL-36R-IL-23-03 light chain (SEQ ID NO: 72) 
EVQLVESGGGLVQPGGSLRLSCAASGNTFSRYCMGWFRQAPGKELVGVAAIDS
DGDTSYADSVEGRFTISRDNAKNTLYLQMNSLRAEDTAVYYCAADFTPSAPPR
MGSGGYCFGDYWGQGTLVTVSSGGGGSGGGGSDIVLTQSPDSLAVSLGERATIN
CRASESVDYYGISSMHWFQQKPGQPPKLLIYRASNLESGIPARFSGSGSRTDFTL
TISSLQAEDVAVYYCQQANKDPYTFGGGTKVEIKRTVAAPSVFIFPPSDEQLKSGT
ASVVCLLNNFYPREAKVQWKVDNALQSGNSQESVTEQDSKDSTYSLSSTLTLSKADYE
KHKVYACEVTHQGLSSPVTKSFNRGEC
> IL-36R-IL-23-04 heavy chain
   is set forth in SEQ ID NO: 71.
> IL-36R-IL-23-04 light chain (SEQ ID NO: 73)

EVQLVESGGGLVQPGGSLRLSCAASGYSYRTQCLGWYRQAPGKQREAVATISG
GGTSTYYSDSVKGRFTISQDNSKTTVYLQMNSLRAEDTAVYYCAKGLSVLGSW
CHGGSSYEYTYSGQGTLVTVSSGGGGSGGGGSDIVLTQSPDSLAVSLGERATINC
RASESVDYYGISSMHWFQQKPGQPPKLLIYRASNLESGIPARFSGSGSRTDFTLTI
SSLQAEDVAVYYCQQANKDPYTFGGGTKVEIKRTVAAPSVFIFPPSDEQLKSGTAS
VVCLLNNFYPREAKVQWKVDNALQSGNSQESVTEQDSKDSTYSLSSTLTLSKADYEK
HKVYACEVTHQGLSSPVTKSFNRGEC

**In** addition, YTE mutations (M252Y, S254T, and T256E mutations, according to the EU numbering scheme) were introduced into the Fc of the anti-IL-36R and anti-IL-23 bispecific antibodies IL-36R-IL-23-01 and IL-36R-IL-23-02 to construct IL-36R-IL-23-01-YTE and IL-36R-IL-23-02-YTE molecules.
> IL-36R-IL-23-01-YTE heavy chain (SEQ ID NO: 74) 
EVQLVESGGGLVQPGGSLRLSCAASGNTFSRYCMGWFRQAPGKELVGVAAIDS
DGDTSYADSVEGRFTISRDNAKNTLYLQMNSLRAEDTAVYYCAADFTPSAPPR
MGSGGYCFGDYWGQGTLVTVSSGGGGSGGGGSQVTLKESGPVLVKPTETLTLT
CTVSGFSLTNYAISWVRQPPGKGLEWLGVIWTGGGTNYNSGIKSRLTISKDNSKS
QVVLTMTNMDPVDTARYYCVREGDYGSYAGAMDYWGQGTTVTVSSASTKGPS
 
VFPLAPSSKSTSGGTAALGCLVKDYFPEPVTVSWNSGALTSGVHTFPAVLQSSGLYSLS
SVVTVPSSSLGTQTYICNVNHKPSNTKVDKKVEPKSCDKTHTCPPCPAPEAAGGPSVF
LFPPKPKDTLYITREPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRE
EMTKNQVSLTCLVKGFYPSDIAVEWESNGQPENNYKTTPPVLDSDGSFFLYSKLTVD
KSRWQQGNVFSCSVMHEALHNHYTQKSLSLSPGK
> IL-36R-IL-23-02-YTE heavy chain (SEQ ID NO: 75) 
EVQLVESGGGLVQPGGSLRLSCAASGYSYRTQCLGWYRQAPGKQREAVATISG
GGTSTYYSDSVKGRFTISQDNSKTTVYLQMNSLRAEDTAVYYCAKGLSVLGSW
CHGGSSYEYTYSGQGTLVTVSSGGGGSGGGGSQVTLKESGPVLVKPTETLTLTC
TVSGFSLTNYAISWVRQPPGKGLEWLGVIWTGGGTNYNSGIKSRLTISKDNSKS
QVVLTMTNMDPVDTARYYCVREGDYGSYAGAMDYWGQGTTVTVSSASTKGPS
VFPLAPSSKSTSGGTAALGCLVKDYFPEPVTVSWNSGALTSGVHTFPAVLQSSGLYSLS
SVVTVPSSSLGTQTYICNVNHKPSNTKVDKKVEPKSCDKTHTCPPCPAPEAAGGPSVF
LFPPKPKDTLYITREPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRE
EMTKNQVSLTCLVKGFYPSDIAVEWESNGQPENNYKTTPPVLDSDGSFFLYSKLTVD
KSRWQQGNVFSCSVMHEALHNHYTQKSLSLSPGK
> IL-36R-IL-23-01-YTE and IL-36R-IL-23-02-YTE light chains are all set forth in SEQ ID NO: 69.
> IgG1 Fc (comprising L234A/L235A/M252Y/S254T/T256E mutations) (SEQ ID NO: 76)

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPEAAGGPSVFLFPPKPKDTLYITREPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPE
NNYKTTPPVLDSDGSFFLYSKLTVDKSRWQQGNVFSCSVMHEALHNHYTQKSL
SLSPGK

### Example 7. Affinity of Anti-IL-36R and Anti-IL-23 Bispecific Antibodies

### 1. Binding to IL-23 (ELISA)

To assess the binding of the anti-IL-36R and anti-IL-23 bispecific antibodies to the IL-23 antigen, the binding of the anti-IL-36R and anti-IL-23 bispecific antibodies to the IL-23 antigen was detected by ELISA. A human IL-23 protein (ACROBiosystems, ILB-H52W5) was dissolved in PBS to 2 µg/mL, and the solution was added to a 96-well plate at 100 µL/well. The plate was coated with the antigen overnight at 4 °C. The plate was washed with a PBST (PBS + 0.05% tween20) solution three times. A blocking solution (PBST/1% BSA solution) was added at 200 µL/well, and the plate was incubated at 37 °C for 1 h for blocking. After three washes with a PBST solution, the anti-IL-23 monoclonal antibodies or the anti-IL-36R and anti-IL-23 bispecific antibodies at different concentrations (diluted with a blocking solution) were added, and the plate was incubated at 37 °C for 2 h. The plate was washed with a PBST solution three times. An HRP-coupled anti-human IgG Fc secondary antibody (100 µL, 1:10000, GenScript, A01854) solution was added, and the plate was incubated at 37 °C for 1 h. After three washes with a PBST solution, a TMB solution was added at 100 µL/well. After 5-10 min of reaction at room temperature, 100 µL of a stop solution was added. OD450 values were measured using a multi-mode microplate reader, and EC₅₀ was calculated.

The anti-human IL-23 antibody risankizumab from AbbVie, the anti-human IL-23 antibody guselkumab from Johnson & Johnson, and the full-length antibody Hu29-19 (SEQ ID NOs: 62 and 63) were used as controls.
> Guselkumab heavy chain (SEQ ID NO: 77) 
EVQLVQSGAEVKKPGESLKISCKGSGYSFSNYWIGWVRQMPGKGLEWMGIIDP
SNSYTRYSPSFQGQVTISADKSISTAYLQWSSLKASDTAMYYCARWYYKPFDV
WGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKV
EPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPSDI
AVEWESNGQPENNYKTTPPVLDSDGSFFLYSKLTVDKSRWQQGNVFSCSVMHE
ALHNHYTQKSLSLSPG
> Guselkumab light chain (SEQ ID NO: 78)

QSVLTQPPSVSGAPGQRVTISCTGSSSNIGSGYDVHWYQQLPGTAPKLLIYGNSK
RPSGVPDRFSGSKSGTSASLAITGLQSEDEADYYCASWTDGLSLVVFGGGTKLT
VLGQPKAAPSVTLFPPSSEELQANKATLVCLISDFYPGAVTVAWKADSSPVKAG
VETTTPSKQSNNKYAASSYLSLTPEQWKSHRSYSCQVTHEGSTVEKTVAPTECS
As shown in FIG. 6A and Table 13, the binding affinities of H[?]36D[?]H[?]30.01 and H

As shown in FIG. 6A and Table 13-1, the binding affinities of IL-36R-IL-23-01 and IL-36R-1L-23-02 for the human IL-23 antigen were comparable to that of Hu29-19 and higher than those of the control antibodies risankizumab and guselkumab.

**Table 13-1. EC₅₀ values for binding of bispecific antibodies to human IL-23 measured by ELISA**

| Antibody No. | Binding activity |
|---|---|
| | Human EC₅₀ (nM) |
| IL-36R-IL-23-01 | 1.21 |
| IL-36R-IL-23-02 | 0.49 |
| Hu29-19 | 0.55 |
| Risankizumab | 4.72 |
| Guselkmab | 3.16 |

### 2. Binding to IL-23 (SPR)

The affinities of the anti-IL-36R and anti-IL-23 bispecific antibodies for the IL-23 antigen were measured by SPR. Each test monoclonal antibody or bispecific antibody was used as a ligand, and the human IL-23 antigen protein (ACROBiosystems, ILB-H52W5) was used as an analyte. The assay was performed using the same experimental method as in Example 3. The results are shown in Table 13-2.

**Table 13-2. Affinities of bispecific antibodies for human IL-23 antigen by SPR**

| Antibody No. | Antigen | ka (1/Ms) | **Kd** (1/s) | K_{D} (M) |
|---|---|---|---|---|
| IL-36R-IL-23-01 | Human IL-23 antigen | 2.60E+06 | 5.97E-05 | 2.30E-11 |
| IL-36R-IL-23-02 | Human IL-23 antigen | 2.90E+06 | 6.83E-05 | 2.35E-11 |
| IL-36R-IL-23-03 | Human IL-23 antigen | 9.54E+05 | 4.93E-05 | 5.17E-11 |
| IL-36R-IL-23-04 | Human IL-23 antigen | 8.58E+05 | 6.02E-05 | 7.02E-11 |
| Hu29-19 | Human IL-23 antigen | 5.93E+06 | 6.26E-05 | 1.06E-11 |

The results show that each bispecific antibody has a relatively strong affinity for the human IL-23 antigen. The affinities of IL-36R-IL-23-01 and IL-36R-IL-23-02 for the human IL-23 antigen were similar to that of Hu29-19.

### 3. Binding to IL-36R on cell surface (FACS)

The binding ability of the anti-IL-36R and anti-IL-23 bispecific antibodies to human IL-36R expressed on the cell membrane was determined by FACS. The experimental procedures were as follows: Candidate test antibodies at different concentrations were added to CHO-K1 cells (1E5 cells/well) overexpressing human IL-36R, and the cells were incubated on ice for 1 h. After the cells were washed twice with a FACS buffer (1% FBS, DPBS), 100 µL of fluorescent secondary antibody AF488-goat anti-human IgG (H + L) (1:1000, Invitrogen, A11013) was added, and the cells were incubated on ice for 30 min. The cells were washed twice with a FACS buffer and resuspended in 150 µL of a FACS buffer. Fluorescence signals were detected on a flow cytometer (BD, FACSCelesta), and the EC₅₀ was calculated.

The results are shown in FIG. 6B and Table 13-3. IL-36R-IL-23-01 and IL-36R-IL-23-02 have relatively strong binding activity to human IL-36R expressed on the surface of CHO-K1 cells, and have similar binding affinities to VHH213_Hu07 and VHH162_Hu11 as well as control antibodies BI655130 and H4H14706P2.

**Table 13-3. EC₅₀ values for binding of bispecific antibodies to human IL-36R measured by FACS**

| Antibody No. | Binding activity | |
|---|---|---|
| | Human EC₅₀ (nM) | Maximum MFI |
| IL-36R-IL-23-01 | 1.28 | 11564 |
| IL-36R-IL-23-02 | 1.13 | 11361 |
| VHH213_Hu07 | 0.97 | 7401 |
| VHH162_Hu11 | 0.88 | 6211 |
| BI655130 | 0.75 | 12893 |
| H4H14706P2 | 0.64 | 10627 |

### 4. Binding to IL-36R (SPR)

The affinities of the anti-IL-36R and anti-IL-23 bispecific antibodies for the IL-36R antigen were measured by SPR. Each test bispecific antibody was used as a ligand, and the human IL-36R antigen protein (Kactus, IL1-HM4L2) was used as an analyte. The experimental procedures were as follows: Biacore^{™} 8k (GE Healthcare) was used. In the experiment, a CM5 sensor chip (Cat. # 29149603, GE) was used, and an HBS-EP + buffer (10 mM HEPES, 150 mM NaCl, 3 mM EDTA, 0.05% surfactant P20) was used as a mobile phase. A 30 µg/mL solution of anti-human IgG (Fc) antibody was prepared in a 10 mM sodium acetate buffer (pH 5.0), and the Immobilization program was selected to automatically perform anti-human IgG (Fc) antibody channel amino coupling immobilization. The test antibodies were separately diluted in an HBS-EP + buffer as ligands and captured using the anti-human IgG (Fc) antibody on the chip channels. The human IL-36R antigen protein (Kactus, IL1-HM4L2) was used as an analyte. The analyte was serially diluted 2-fold with an HBS-EP + buffer, and the dilution was made to flow through the experimental and reference channels at a flow rate of 30 µL/min, with association for 1 min and dissociation for 15 min. The regeneration buffer 10 mM Glycine pH 1.5 (GE Healthcare, BR-1003-54) was run at a flow rate of 10 µL/min for 30 s. The association rate Ka, the dissociation rate Kd, and the dissociation constant (i.e., avidity K_{D}) were calculated. The results are shown in Table 13-4.

**Table 13-4. Affinities of bispecific antibodies for IL-23 antigen by SPR**

| Antibody No. | Antigen | Ka (1/Ms) | Kd (1/s) | K_{D} (M) |
|---|---|---|---|---|
| IL-36R-IL-23-01 | Human IL-36R antigen | 1.91E+04 | 1.23E-04 | 6.47E-09 |
| IL-36R-IL-23-02 | Human IL-36R antigen | 9.21E+03 | 1.46E-04 | 1.59E-08 |
| IL-36R-IL-23-03 | Human IL-36R antigen | 2.53E+04 | 1.08E-04 | 4.28E-09 |
| IL-36R-IL-23-04 | Human IL-36R antigen | 1.22E+04 | 2.16E-04 | 1.77E-08 |

The results show that each bispecific antibody has a relatively strong affinity for the human IL-36R antigen, and has a similar affinity to VHH162_Hu11 and VHH213_Hu07 for the human IL-36R antigen.

### Example 8. Inhibitory Effects of Anti-IL-36R and Anti-IL-23 Bispecific Antibodies on IL-36 Stimulating Factor Activation of IL-36R Function

The inhibitory activity of the anti-IL-36R and anti-IL-23 bispecific antibodies against IL-36-induced IL-8 secretion was evaluated using the human skin squamous cell line A431. The experimental procedures were as follows: A431 cells (ATCC, HRC00439) were plated on a 96-well plate at 3E4 cells/well and cultured overnight. Diluted test antibodies at different concentrations and IL-36 (IL36α (CR61), IL36β (CR59), or IL36γ (CM77), NOVOProtein) were added to each well. After 24 h of culture, the supernatant was collected and assayed for IL-8 content using an IL-8 kit (Cisbio, 62HIL08PEG) and a multi-mode microplate reader (Perkin Elmer, Envision2105).

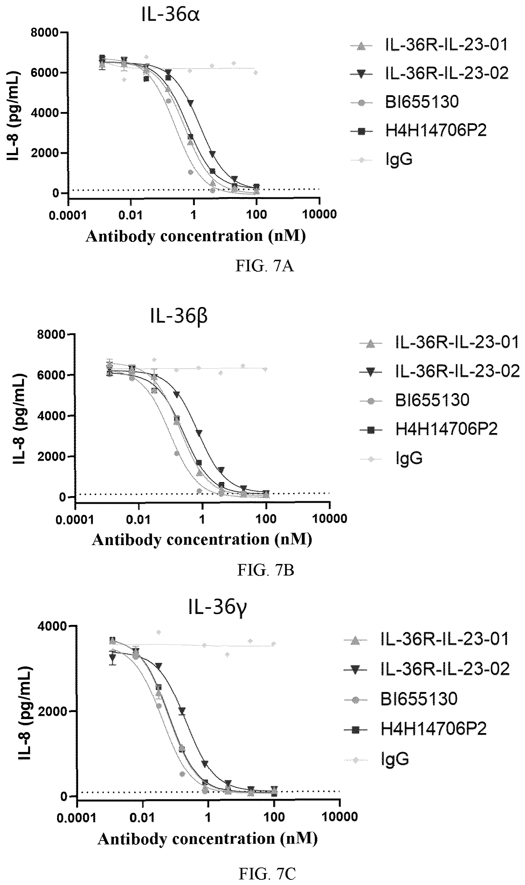

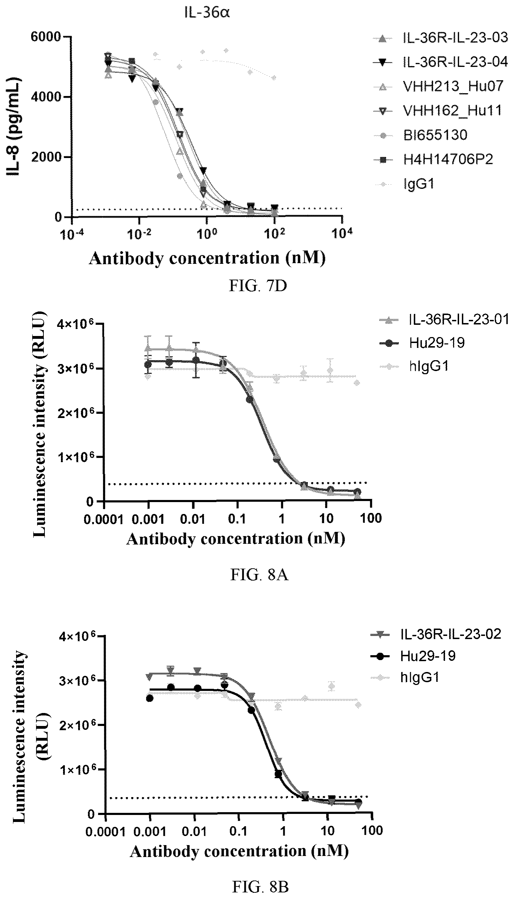

The results are shown in FIGs. 7A and 7B and Tables 14-1 and 14-2. IL-36R-IL-23-01, IL-36R-IL-23-02, IL-36R-IL-23-03, and IL-36R-IL-23-04 can significantly block the secretion of IL-8 by A431 induced by human IL-36.

**Table 14-1. Inhibitory effects of IL-36R-IL-23-01 and IL-36R-IL-23-02 on human IL-36 stimulating factor function**

| Antibody No. | IL-8 secretion (A431) | | |
|---|---|---|---|
| | IL-36α | IL-36β | IL-36γ |
| | IC₅₀ (nM) | IC₅₀ (nM) | IC₅₀ (nM) |
| IL-36R-IL-23-01 | 0.54 | 0.2 | 0.06 |
| IL-36R-IL-23-02 | 1.63 | 0.74 | 0.21 |
| BI655130 | 0.26 | 0.1 | 0.04 |
| H4H14706P2 | 0.64 | 0.26 | 0.06 |

**Table 14-2. Inhibitory effects of IL-36R-IL-23-03 and IL-36R-IL-23-04 on human IL-36 stimulating factor function**

| Antibody No. | IL-36α-induced A431 IL-8 secretion IC₅₀ (nM) |
|---|---|
| IL-36R-IL-23-03 | 0.29 |
| IL-36R-IL-23-04 | 0.34 |
| VHH213_Hu07 | 0.13 |
| VHH162_Hu11 | 0.14 |
| BI655130 | 0.06 |
| H4H14706P2 | 0.15 |

### Example 9. Inhibitory Effects of Anti-IL-36R and Anti-IL-23 Bispecific Antibodies on IL-23 Stimulating Factor Activation of IL-23R Function

### 1. Inhibitory effects on proliferation of Ba/F3-IL-23 cells induced by human IL-23

The inhibitory effects of the anti-IL-36R and anti-IL-23 bispecific antibodies on the proliferation of BaF3-mIL-23R cells induced by the human IL-23 protein were evaluated by the CellTiter Glo method. BaF3-mIL-23R cells (BaF3 cells (Cobioer, CBP60474) stably expressing IL-23R and IL-12Rβ1) were plated in a 96-well plate at 1E4 cells/well (50 µL); 25 µL of each of the anti-IL-36R and anti-IL-23 bispecific antibodies at different concentrations was incubated with 25 µL of human IL-23 (ACROBiosystems, ILB-H52W5, final concentration: 150 ng/mL) for half an hour; and then the mixture was added to the cell culture plate. After 72 h of incubation, 50 µL of a Celltiter Glo detection solution (Promega, G7573) was added to each well, and bioluminescent signals were detected using a multi-mode microplate reader (Perkin Elmer, Envision 2105).

The results are shown in FIGs. 8A and 8B and Tables 15-1 and 15-2. IL-36R-IL-23-01 and IL-36R-IL-23-02 significantly inhibit the proliferation of BaF3-mIL-23R cells induced by the human IL-23 protein.

**Table 15-1. Inhibitory effects of IL-36R-IL-23-01 on proliferation of BaF3-mIL-23R cells induced by IL-23**

| Antibody No. | IC₅₀ (nM) |
|---|---|
| IL-36R-IL-23-01 | 0.39 |
| Hu29-19 | 0.36 |

**Table 15-2. Inhibitory effects of IL-36R-IL-23-02 on proliferation of BaF3-mIL-23R cells induced by IL-23**

| Antibody No. | IC₅₀ (nM) |
|---|---|
| IL-36R-IL-23-02 | 0.5 |
| Hu29-19 | 0.43 |

### 2. Inhibitory effects on human IL-23-induced STAT3 activation signal of DB-STAT3-Luc2 reporter gene system

The inhibitory effects of the anti-IL-36R and anti-IL-23 bispecific antibodies on the human IL-23 protein-induced STAT3 activation signal in DB-STAT3 luc2 cells were evaluated using a luciferase reporter system.

DB-STAT3-Luc2 cells (a DB cell strain stably expressing the STAT3-Luc2 reporter gene system (ATCC, CRL-2289)) were plated in a 96-well plate at 3E4 cells/well (50 µL); 25 µL of each of the anti-IL-36R and anti-IL-23 bispecific antibodies at different concentrations was incubated with 25 µL of human IL-23 (ACROBiosystems, ILB-H52W5, final concentration: 1 µg/mL) for half an hour; and then the mixture was added to the cell culture plate. After overnight incubation, 100 µL of a Bright-Glo (Promega, E2620) detection solution was added to each well, and bioluminescent signals were detected using a multi-mode microplate reader (Perkin Elmer, Envision 2105).

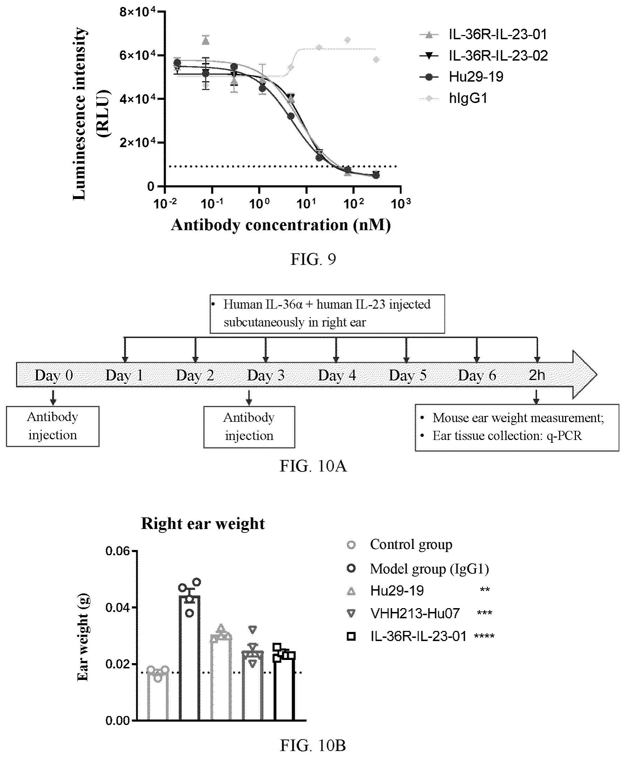

The results are shown in FIG. 9 and Table 16. The results show that in the DB-STAT3-Luc2 reporter gene system, the anti-IL-36R and anti-IL-23 bispecific antibodies IL-36R-IL-23-01 and IL-36R-IL-23-02 significantly inhibit the human IL-23 protein-induced STAT3 signal activation, and the inhibitory effects are comparable to that of the anti-IL-23 monoclonal antibody HU29-19.

**Table 16. Inhibitory effects of bispecific antibodies on IL-23-induced DB-STAT3-Luc2 reporter gene system**

| Antibody No. | DB-STAT3-Luc2 reporter gene system IC₅₀ (nM) |
|---|---|
| IL-36R-IL-23-01 | 7 |
| IL-36R-IL-23-02 | 8.8 |
| Hu29-19 | 5.1 |

### Example 10. Inhibitory Effects of Bispecific Antibody on Mouse Ear Swelling Induced by Human IL-36 and Human IL-23

This model was used to simulate inflammatory diseases caused by excessive IL-36 or IL-23 in humans.

Female human IL-36R transgenic mice aged 6-8 weeks were purchased from Biocytogen Jiangsu Co., Ltd. Housing environment: SPF; production license: SCXK(Jiangsu)-2021-0003; human IL-36R transgenic mouse certification number: 320726210100341485.

### 1. Inhibitory effects of bispecific antibody in mouse acute ear swelling model

A mouse acute ear swelling model was constructed by subcutaneous injection of human IL-36 and human IL-23 into the ears of IL-36R humanized transgenic mice to evaluate the *in vivo* activity of the anti-IL-36R and anti-IL-23 bispecific antibody.

The experimental process is shown in FIG. 10A. After arrival, the animals were acclimatized for 7 days and randomly grouped. On day 0 and day 3 of the experiment, the mice were intraperitoneally injected with an equimolar amount of the anti-IL-36R antibody, the anti-IL-23 antibody, or the anti-IL-36R and anti-IL-23 bispecific antibody. From day 1 to day 6 of the experiment, PBS or 1 µg of a recombinant human IL-36α protein and 3 µg of a recombinant human IL-23 protein were subcutaneously injected into the right ear of the mice daily, and an equal volume of PBS was injected into the left ear. The mouse grouping and administration regimens are shown in Table 17. The right ear weight of the mice was measured at the end of the experiment, and part of the ear tissue was taken and stored with RNAlater (Invitrogen, AM7020) for downstream gene expression detection.

**Table 17. Administration regimens for human IL-36R transgenic mouse ear swelling induced by human IL-36 and human IL-23**

| Mouse Strain | Group | N | Left ear | Right ear | Administration | Dose | Route of administration |
|---|---|---|---|---|---|---|---|
| Human IL-36R transgenic mice C57BL/6 | 1 | 3 | PBS | PBS | / | / | ip |
| | 2 | 4 | PBS | hIL-36α 1µg + hIL-23 3µg | IgG | 10 mg/kg | ip |
| | 3 | 4 | PBS | | Hu29-19 | 10 mg/kg | ip |
| | 4 | 5 | PBS | | VHH213-Hu07 | 5.3 mg/kg | ip |
| | 5 | 5 | PBS | | IL-36R-IL-23-01 | 12 mg/kg | ip |

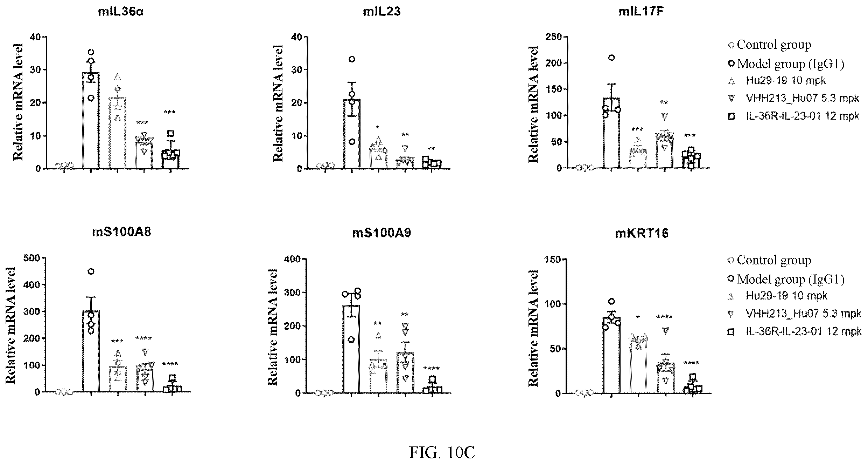

The results are shown in FIGs. 10B and 10C. The bispecific antibody IL-36R-IL-23-01 significantly inhibits the increase in the right ear weight of the mice induced by human IL-36 and human IL-23 (FIG. 10B) and reduces the expression levels of inflammatory disease factors and antimicrobial peptides (FIG. 10C). IL-36R-IL-23-01 has a stronger regulatory effect on the downstream gene expression of the ear tissues than the anti-IL-36R antibody VHH213_Hu07 and the anti-IL-23 antibody HU29-19 (FIG. 10C) (in the figure, as compared to the model group, * P < 0.05, ** P < 0.01, *** P < 0.001, and **** P < 0.0001).

### 2. Inhibitory effects of bispecific antibody in mouse acute ear swelling model as compared to monoclonal antibody combination

The experimental process is shown in FIG. 10A. After arrival, the animals were acclimatized for 7 days and randomly grouped. On day 0 of the experiment, the mice were intraperitoneally injected with an equimolar amount of the anti-IL-36R antibody, the anti-IL-23 antibody, the anti-IL-36R and anti-IL-23 bispecific antibody, or the anti-IL-36R antibody in combination with the anti-IL-23 antibody. From day 1 to day 6 of the experiment, PBS or 1 µg of a recombinant human IL-36 protein and 3 µg of a recombinant human IL-23 protein were subcutaneously injected into the right ear of the mice daily, and an equal volume of PBS was injected into the left ear. The mouse grouping and administration regimens are shown in Table 18. The right ear weight of the mice was measured at the end of the experiment, and part of the ear tissue was taken and stored with RNAlater (Invitrogen, AM7020) for downstream gene expression detection.

**Table 18. Administration regimens for human IL-36R transgenic mouse ear swelling induced by human IL-36 and human IL-23**

| Mouse Strain | Group | N | Left ear | Right ear | Administration | Dose | Route of administration |
|---|---|---|---|---|---|---|---|
| Human IL-36R transgenic mice C57BL/6 | 1 | 5 | PBS | PBS | / | / | ip |
| | 2 | 5 | PBS | hIL-36α 1µg + hIL-23 3µg | IgG | 10 mg/kg | ip |
| | 3 | 6 | PBS | | Hu29-19 | 10 mg/kg | ip |
| | 4 | 6 | PBS | | VHH213-Hu07 | 5.3 mg/kg | ip |
| | 5 | 6 | PBS | | IL-36R-IL-23-01 | 12 mg/kg | ip |
| | 6 | 6 | PBS | | IL-36R-IL-23-01 | 24 mg/kg | ip |
| | 7 | 6 | PBS | | Hu29-19+ VHH213-Hu07 | 10 mg/kg + 5.3 mg/kg | ip |

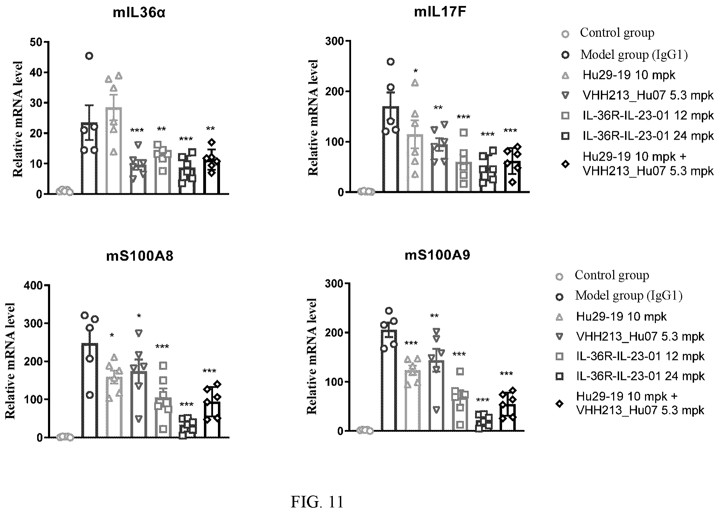

The results are shown in FIG. 11. The bispecific antibody IL-36R-IL-23-01 (12 mpk) has a significantly stronger regulatory effect on the downstream gene expression of the mouse ear tissues than the anti-IL-23 antibody Hu29-19 (10 mpk) and the anti-IL-36R antibody VHH213_Hu07 (5.3 mpk) at an equimolar dose, and the regulatory effect is comparable to the combination group of Hu29-19 (10 mpk) and VHH213_Hu07 (5.3 mpk). In addition, IL-36R-IL-23-01 exhibits a significant dose effect, and the regulatory effect of IL-36R-IL-23-01 at the high dose (24 mpk) on multiple genes (e.g., S100A8 and S100A9, both antibacterial peptides, with up-regulated expression in inflammatory responses and being involved in cytokine recruitment and cytokine expression) is stronger than that of IL-36R-IL-23-01 at the low dose (12 mpk) and the combination of Hu29-19 (10 mpk) and VHH213_Hu07 (5.3 mpk) (in the figure, as compared to the model group, * P < 0.05, ** P < 0.01, and *** P < 0.001).

Although specific embodiments of the present disclosure have been described above, it will be appreciated by those skilled in the art that these embodiments are merely illustrative and that many changes or modifications can be made to these embodiments without departing from the principles and spirit of the present disclosure. The scope of protection of the present disclosure is therefore defined by the claims.

## Claims

1. An IL-36R/IL-23-binding protein, comprising a first antigen-binding domain that specifically binds to IL-36R and a second antigen-binding domain that specifically binds to IL-23,
wherein the first antigen-binding domain comprises an immunoglobulin single variable domain, and the immunoglobulin single variable domain comprises:
a CDR1, a CDR2, and a CDR3 from the amino acid sequence set forth in any one of SEQ ID NOs: 3 and 12-26, or
a CDR1, a CDR2, and a CDR3 from the amino acid sequence set forth in any one of SEQ ID NOs: 4 and 27-35,
wherein the CDR1, CDR2, and CDR3 are defined according to the Kabat, IMGT, Chothia, AbM, or Contact numbering scheme.

2. The IL-36R/IL-23-binding protein according to claim 1, wherein the amino acid sequences of the CDR1, CDR2, and CDR3 of the immunoglobulin single variable domain are set forth in SEQ ID NOs: 5, 6, and 7, or SEQ ID NOs: 8, 9, and 10, respectively.

3. The IL-36R/IL-23-binding protein according to claim 1 or 2, wherein the immunoglobulin single variable domain is engineered by humanization, back mutation, avidity maturation, T cell epitope removal, reduction of antibody deamidation, and/or reduction of antibody isomerization;
preferably, heavy chain framework regions of a human germline template used in the engineering by humanization are derived from IGHV3-23*04, IGHV3-74*01, and/or IGHJ1*01.

4. The IL-36R/IL-23-binding protein according to any one of claims 1-3, wherein the amino acid sequence of the immunoglobulin single variable domain is set forth in any one of SEQ ID NOs: 3 and 12-26 or has at least 80% or at least 90% sequence identity thereto, or is set forth in any one of SEQ ID NOs: 4 and 27-35 or has at least 80% or at least 90% sequence identity thereto;
preferably, the immunoglobulin single variable domain is an anti-IL-36R nanobody or VHH.

5. The IL-36R/IL-23-binding protein according to any one of claims 1-4, wherein the second antigen-binding domain that specifically binds to IL-23 comprises a heavy chain variable region (VH) and a light chain variable region (VL), wherein
the VH comprises a HCDR1, a HCDR2, and a HCDR3 from the amino acid sequence set forth in any one of SEQ ID NOs: 52-57, and the VL comprises a LCDR1, a LCDR2, and a LCDR3 from the amino acid sequence set forth in any one of SEQ ID NOs: 58-61, or the VH comprises a HCDR1, a HCDR2, and a HCDR3 from the amino acid sequence set forth in SEQ ID NO: 40, and the VL comprises a LCDR1, a LCDR2, and a LCDR3 from the amino acid sequence set forth in SEQ ID NO: 41,
wherein the CDRs are defined according to the Kabat, IMGT, Chothia, AbM, or Contact numbering scheme;
preferably,
the VH comprises a HCDR1, a HCDR2, and a HCDR3 of the amino acid sequences set forth in SEQ ID NOs: 50, 43, and 44, and the VL comprises a LCDR1, a LCDR2, and a LCDR3 of the amino acid sequences set forth in SEQ ID NOs: 45-47;
the VH comprises a HCDR1, a HCDR2, and a HCDR3 of the amino acid sequences set forth in SEQ ID NOs: 51, 43, and 44, and the VL comprises a LCDR1, a LCDR2, and a LCDR3 of the amino acid sequences set forth in SEQ ID NOs: 45-47;
the VH comprises a HCDR1, a HCDR2, and a HCDR3 of the amino acid sequences set forth in SEQ ID NOs: 42-44, and the VL comprises a LCDR1, a LCDR2, and a LCDR3 of the amino acid sequences set forth in SEQ ID NOs: 45-47.

6. The IL-36R/IL-23-binding protein according to claim 5, wherein
the VH comprises the amino acid sequence set forth in any one of SEQ ID NOs: 52-57 or an amino acid sequence having at least 80% or at least 90% identity thereto, and the VL comprises the amino acid sequence set forth in any one of SEQ ID NOs: 58-61 or an amino acid sequence having at least 80% or at least 90% identity thereto, or
the VH comprises the amino acid sequence set forth in SEQ ID NO: 40 or an amino acid sequence having at least 80% or at least 90% identity thereto, and the VL comprises the amino acid sequence set forth in SEQ ID NO: 41 or an amino acid sequence having at least 80% or at least 90% identity thereto;
preferably, the VH comprises the amino acid sequence set forth in SEQ ID NO: 56, and the VL comprises the amino acid sequence set forth in SEQ ID NO: 60.

7. An IL-36R/IL-23-binding protein, comprising a first antigen-binding domain that specifically binds to IL-36R and a second antigen-binding domain that specifically binds to IL-23, wherein preferably, the first antigen-binding domain and the second antigen-binding domain are as defined in any one of claims 1-6.

8. The IL-36R/IL-23-binding protein according to any one of claims 1-7, further comprising an Fc region of an immunoglobulin, wherein
preferably, the Fc region is an Fc region of human IgG1, human IgG2, or human IgG4;
more preferably, the Fc region is an Fc region of human IgG4 comprising an S228P mutation, or an Fc region of human IgG1 comprising an L234A and/or L235A mutation, or an Fc region of human IgG1 comprising an M252Y, S254T, and/or T256E mutation.

9. The IL-36R/IL-23-binding protein according to any one of claims 1-8, wherein the binding protein further comprises a linker;
preferably, the amino acid sequence of the linker is represented by (GₘSₙ)ₕ or (GGNGT)ₕ or (YGNGT)ₕ or (EPKSS)ₕ, wherein m and n are each independently selected from the group consisting of an integer of 1-8, and h is independently selected from the group consisting of an integer of 1-20;
more preferably, the linker is a linker of (G₄S)₂ or (G₄S)₃.

10. The IL-36R/IL-23-binding protein according to any one of claims 1-9, comprising a first polypeptide chain and a second polypeptide chain, wherein the first polypeptide chain and the second polypeptide chain are selected from the group consisting of:
(1) a first polypeptide chain comprising the amino acid sequence set forth in SEQ ID NO: 68 or 70 or an amino acid sequence having at least 80% or at least 90% sequence identity thereto, and a second polypeptide chain comprising the amino acid sequence set forth in SEQ ID NO: 69 or an amino acid sequence having at least 80% or at least 90% sequence identity thereto;
(2) a first polypeptide chain comprising the amino acid sequence set forth in SEQ ID NO: 71 or an amino acid sequence having at least 80% or at least 90% sequence identity thereto, and a second polypeptide chain comprising the amino acid sequence set forth in SEQ ID NO: 72 or 73 or an amino acid sequence having at least 80% or at least 90% sequence identity thereto;
(3) a first polypeptide chain comprising the amino acid sequence set forth in SEQ ID NO: 74 or 75 or an amino acid sequence having at least 80% or at least 90% sequence identity thereto, and a second polypeptide chain comprising the amino acid sequence set forth in SEQ ID NO: 69 or an amino acid sequence having at least 80% or at least 90% sequence identity thereto.

11. An IL-36R-binding protein, comprising an immunoglobulin single variable domain, wherein the immunoglobulin single variable domain comprises:
a CDR1, a CDR2, and a CDR3 from the amino acid sequence set forth in any one of SEQ ID NOs: 3 and 12-26, or
a CDR1, a CDR2, and a CDR3 from the amino acid sequence set forth in any one of SEQ ID NOs: 4 and 27-35,
wherein the CDR1, CDR2, and CDR3 are defined according to the Kabat, IMGT, Chothia, AbM, or Contact numbering scheme.

12. The IL-36R-binding protein according to claim 11, wherein the amino acid sequences of the CDR1, CDR2, and CDR3 of the immunoglobulin single variable domain are set forth in SEQ ID NOs: 5, 6, and 7, or SEQ ID NOs: 8, 9, and 10, respectively.

13. The IL-36R-binding protein according to claim 11 or 12, wherein the immunoglobulin single variable domain is engineered by humanization, back mutation, avidity maturation, T cell epitope removal, reduction of antibody deamidation, and/or reduction of antibody isomerization;
preferably, heavy chain framework regions of a human germline template used in the engineering by humanization are derived from IGHV3-23*04, IGHV3-74*01, and/or IGHJ1*01.

14. The IL-36R-binding protein according to any one of claims 11-13, wherein the amino acid sequence of the immunoglobulin single variable domain is set forth in any one of SEQ ID NOs: 3 and 12-26 or has at least 80% or at least 90% sequence identity thereto, or is set forth in any one of SEQ ID NOs: 4 and 27-35 or has at least 80% or at least 90% sequence identity thereto;
preferably, the IL-36R-binding protein is an anti-IL-36R nanobody or VHH.

15. The IL-36R-binding protein according to any one of claims 11-14, further comprising an Fc region of an immunoglobulin, wherein
preferably, the Fc region is an Fc region of human IgG1, human IgG2, or human IgG4.

16. A polynucleotide, wherein the polynucleotide encodes the IL-36R/IL-23-binding protein according to any one of claims 1-10 or the IL-36R-binding protein according to any one of claims 11-15;
preferably, the polynucleotide is a DNA or an RNA.

17. A vector, comprising the polynucleotide according to claim 16.

18. A host cell, comprising or expressing the polynucleotide according to claim 16 or the vector according to claim 17.

19. A method for preparing an IL-36R/IL-23-binding protein or an IL-36R-binding protein, comprising:
expressing the polynucleotide according to claim 16 or the vector according to claim 17 in the host cell according to claim 18, and isolating an expressed IL-36R/IL-23-binding protein or IL-36R-binding protein from the host cell,
wherein optionally, the method further comprises a step of purifying the IL-36R/IL-23-binding protein or the IL-36R-binding protein.

20. A pharmaceutical composition, comprising the IL-36R/IL-23-binding protein according to any one of claims 1-10, the IL-36R-binding protein according to any one of claims 11-15, and at least one pharmaceutically acceptable excipient, diluent, or carrier.

21. A method for treating or preventing a disease, comprising administering to a subject in need thereof a therapeutically or prophylactically effective amount of the IL-36R/IL-23-binding protein according to any one of claims 1-10, the IL-36R-binding protein according to any one of claims 11-15, the polynucleotide according to claim 16, the vector according to claim 17, or the pharmaceutical composition according to claim 20, wherein preferably, the disease is an inflammatory disease or an autoimmune disease;
more preferably, the disease is psoriasis.

22. Use of the IL-36R/IL-23-binding protein according to any one of claims 1-10, the IL-36R-binding protein according to any one of claims 11-15, the polynucleotide according to claim 16, the vector according to claim 17, or the pharmaceutical composition according to claim 20 in the manufacture of a medicament for treating or preventing a disease, wherein
preferably, the disease is an inflammatory disease or an autoimmune disease;
more preferably, the disease is psoriasis.
